# EUROPEAN PATENT SPECIFICATION

(11) **EP 4 534 138 B1**
(45) Date of publication and mention of the grant of the patent: **25.02.2026**
(21) Application number: 24199107.4
(22) Date of filing: 09.09.2024
(51) Int. Cl.: A61N 1/365, A61B 5/00, A61N 1/368, A61N 1/37, A61N 1/372, A61N 1/375

(54) **DUAL CHAMBER LEADLESS PACEMAKER SYSTEMS**
ZWEIKAMMER-HERZSCHRITTMACHERSYSTEME OHNE ELEKTRODEN
SYSTÈMES DE STIMULATEUR CARDIAQUE SANS FIL À DOUBLE CHAMBRE

(30) Priority: 03.10.2023 US 202363587508 P; 29.08.2024 US 202418819947
(43) Date of publication of application: 09.04.2025
(60) Divisional application: 25223399.4
(73) Proprietor: Pacesetter, Inc., Sylmar, CA 91342 (US)
(72) Inventor: Fishler, Matthew G, Scotts Valley, CA (US); Yang, Weiqun, Cupertino, CA (US); Rogan, Laura L, Thousand Oaks, CA (US); Graumann, Robert J, Jacksonville, FL (US); Wiley IV, Joseph James, Helena, AL (US)
(74) Representative: Barker Brettell Sweden AB

(56) References cited:
- EP-A1- 2 471 448
- US-A1- 2008 255 628
- US-A1- 2020 215 341
- US-A1- 2021 370 078

## Description

### FIELD OF TECHNOLOGY

Embodiments described herein generally relate dual chamber leadless pacemaker (LP) systems.

### BACKGROUND

Conventional pacemakers typically include a housing (aka "can"), which houses a controller (e.g., processor), and one or more intravascular leads that extend from the housing. Each of the leads includes one or more electrodes that can be used for sensing cardiac electrical activity and for delivering pacing stimulation. Such conventional pacemakers can support single-chamber operating modes (e.g., VVI, AAI) and coordinated dual chamber operating modes (e.g., DDD, VDD, DDI, VDI, or DOO), depending upon how many leads are used and how the conventional pacemakers are programmed. It is noted that when such a pacemaker is supporting an operating mode, it can equivalently be said that the pacemaker is providing a type of operation. For example, saying a pacemaker is supporting a VVI operating mode is the same as saying the pacemaker is providing the VVI operation. It is also noted that the terms operating mode and pacing mode are often used interchangeably. For example, saying a pacemaker is operating in a VVI pacing mode is the same as saying the pacemaker is providing the VVI operation, or saying the pacemaker is supporting VVI operation.

Over the past few years, physicians have started implanting leadless pacemakers (LPs) in place of conventional pacemakers because LPs eliminate the need for implanting leads. This is beneficial because leads may fail and/or migrate more often than desired. Initially an LP was only implanted in a single cardiac chamber, e.g., the right ventricular (RV) chamber, and was only capable of being used to support single-chamber operating modes (e.g., VVI).

There is now a desire to start implanting LPs in two cardiac chambers, e.g., in the RV chamber and in the right atrial (RA) chamber, to provide for a dual chamber LP system that is capable of supporting coordinated dual chamber operating modes (e.g., DDD, VDD, DDI, VDI, or DOO). An LP that is implanted in (or on), or configured to be implanted in (or on), the RV chamber may be referred to herein as a ventricular LP, or a vLP. An LP that is implanted in (or on), or configured to be implanted in (or on), the RA chamber may be referred to herein as an atrial LP, or an aLP.

In order for a dual chamber LP pacing system to provide for coordinated dual chamber operation, e.g., of the RV chamber and the RA chamber, the aLP and the vLP may coordinate their activities via beat-to-beat implant-to-implant (i2i) communication, directly with one another, or via a third implantable medical device (IMD) that acts as a communication hub for the LPs. Such beat-to-beat i2i communication between the aLP and the vLP can be achieved using conductive communication, where the same electrodes that an LP uses for delivering pacing pulses are also used for transmitting i2i communication pulses to another LP, as well for receiving i2i communication pulses from the other LP. Such beat-to-beat i2i communication between the aLP and the vLP can alternatively be achieved using another type of communication besides conductive communication, such as radio frequency (RF) communication or inductive communication. Regardless of the type of i2i communication that is used, beat-to-beat i2i communication (i.e., i2i communication that occurs each and every cardiac cycle) consumes a substantial amount of power, and thus, reduces the longevity of the aLP and the vLP by depleting their respective power sources (e.g., batteries) thereof faster than would occur if i2i communication were not used at all, or were used less frequently than every beat. For example, where conductive i2i communication is used on a beat-by-beat basis, the overall longevity of an LP may be significantly reduced compared to if no conductive i2i communication were used by the LP.

US 2021/370078 discloses methods for use with an implantable system including at least an atrial leadless pacemaker (aLP). The aLP senses a signal from which cardiac activity associated with a ventricular chamber can be detected by the aLP itself based on feature(s) of the sensed signal. The aLP monitors the sensed signal for an intrinsic or paced ventricular activation within a ventricular event monitor window. In response to the aLP detecting an intrinsic or paced ventricular activation itself from the sensed signal within the ventricular event monitor window, the aLP resets an atrial escape interval timer that is used by the aLP to time delivery of an atrial pacing pulse if an intrinsic atrial activation is not detected within an atrial escape interval.

US 2008/255628 discloses device comprising a cardiac contraction sensing circuit, a timer circuit, an electrical stimulation circuit, and a controller. The timer circuit provides a time duration of an atrial-atrial interval between successive atrial contractions, a ventricular-ventricular interval between successive ventricular contractions, and an atrial-ventricular (A-V) interval between an atrial contraction and a same cardiac cycle ventricular contraction. The controller includes an event detection module and a pacing module. The event detection module is configured for determining whether A-V block events are sustained over multiple cardiac cycles. The pacing module is configured for providing pacing therapy according to a primary pacing mode that includes AAI(R) mode with independent VVI backup mode, and for switching the pacing therapy to a secondary pacing mode if A-V block events are sustained over multiple cardiac cycles.

### SUMMARY

The invention relates to a dual chamber LP system, comprising an atrial leadless pacemaker (aLP) and a ventricular leadless pacemaker (vLP). The aLP is configured to be implanted in or on an atrial cardiac chamber of a patient's heart. The vLP is configured to be implanted in or on a ventricular cardiac chamber of the patient's heart. The aLP is configured to provide AAI operation, and the vLP is configured to provide VVI operation, during a same period of time and independent of one another. While the aLP provides the AAI operation, the aLP performs atrial pacing when an intrinsic atrial event is not detected within a specified AA interval following a previous paced or intrinsic atrial event, performs atrial sensing, and inhibits the atrial pacing when the intrinsic atrial event is detected within the specified AA interval following the previous paced or intrinsic atrial event. While the vLP provides the VVI operation, the vLP performs ventricular pacing when an intrinsic ventricular event is not detected within a specified VV interval following a previous paced or intrinsic ventricular event, performs ventricular sensing, and inhibits the ventricular pacing when the intrinsic ventricular event is detected within the specified VV interval following the previous paced or intrinsic ventricular event.

In accordance with the invention, each LP, of the aLP and the vLP, includes a respective transmitter that enables the LP to transmit i2i messages, includes a respective receiver that enables the LP to receive i2i messages, and includes a respective power source (e.g., battery) used to power the transmitter and the receiver of the LP. During the same first period of time that the aLP provides the AAI operation and the vLP provides the VVI operation independent of one another, the aLP is configured to abstain from transmitting any i2i messages that are intended to be used to coordinate operation of the vLP with the aLP, and the vLP is configured to abstain from transmitting any i2i messages to that are intended to be used to coordinate operation of the aLP with the vLP.

In accordance with certain embodiments, the specified AA interval is shorter than the specified VV interval to cause an atrial rate of the atrial pacing performed by the aLP to be faster than a ventricular rate of the ventricular pacing performed by the vLP.

In accordance with certain embodiments, the aLP includes a sensor that enables the aLP to monitor an activity level of a patient within which the aLP and the vLP are implanted. Further, each LP, of the aLP and the vLP, includes a respective controller. In certain such embodiments, the controller of the aLP is configured to adjust an atrial rate of the atrial pacing performed by the aLP by adjusting the specified AA interval based on the activity level of the patient as detected using the sensor of the aLP while the aLP is providing the AAI operation. Additionally, the controller of the vLP is configured to not adjust a ventricular rate of the ventricular pacing performed by the vLP while the vLP is providing the VVI operation. In other embodiments, the controller of the aLP is configured to adjust an atrial rate of the atrial pacing performed by the aLP by adjusting the specified AA interval based on the activity level of the patient as detected using the sensor of the aLP while the aLP is providing the AAI operation, and the controller of the vLP is configured to adjust a ventricular rate of the ventricular pacing performed by the vLP by adjusting the VV interval based on the activity level of the patient as detected using the sensor of the vLP while the vLP is providing the VVI pacing, wherein such an embodiment can be referred to more specifically as AAIR+VVIR operation. In such embodiments, the vLP includes a sensor that enables the vLP to monitor the activity level of the patient.

In accordance with certain embodiments, the aLP includes a sense amplifier configured to sense an atrial electrogram (aEGM) used by the aLP to detect intrinsic atrial events, and the vLP includes a sense amplifier configured to sense a ventricular electrogram (vEGM) used by the vLP to detect intrinsic ventricular events. Additionally, the aLP includes a controller that is communicatively coupled to the sense amplifier of the aLP, and the vLP includes a controller that is communicatively coupled to the sense amplifier of the vLP. In certain such embodiments, the controller of the vLP is configured to monitor for possible crosstalk that may be caused by the aLP performing the atrial pacing, while the vLP is providing the VVI operation, and initiate providing crosstalk protection for a first crosstalk protection period, in response to the controller of the vLP detecting possible crosstalk that may be caused by the aLP performing the atrial pacing. Additionally, or alternatively, the controller of the aLP is configured to monitor for possible crosstalk that may be caused by the vLP performing the ventricular pacing, while the aLP is providing the AAI operation, and initiate providing crosstalk protection for a second crosstalk protection period, in response to the controller of the aLP detecting possible crosstalk that may be caused by the vLP performing the ventricular pacing.

In accordance with certain embodiments, each LP, of the aLP and the vLP, includes a respective transmitter that enables the LP to transmit i2i messages to the other LP, includes a respective receiver that enables the LP to receive i2i messages from the other LP, and includes a respective battery used to power the transmitter and the receiver of the LP. Additionally, the aLP includes a controller that is communicatively coupled to the transmitter and the receiver thereof and is powered by the battery thereof, and the vLP includes a controller that is communicatively coupled to the transmitter and the receiver thereof and is powered by the battery thereof. In certain such embodiments, the controller of the vLP is configured to determine whether or not a first specified criterion is satisfied, abstain from using the transmitter of the vLP to transmit any i2i messages that are intended to be used to coordinate operation of the aLP with the vLP during a first period of time when the first specified criterion is not satisfied, and cause the transmitter of the vLP to transmit a mode switch type of i2i message in response to the first specified criterion being satisfied. The mode switch type of i2i message, if successfully received by and acknowledged by the aLP, causes the aLP and the vLP to transition (from respectively providing the AAI operation and the VVI operation, independent of one another) to collectively providing coordinated dual chamber operation during a second period of time, during which i2i messages are transmitted by the aLP and the vLP (to support the coordinated dual chamber operation). In certain embodiments, collectively providing coordinated dual chamber operation, during which i2i messages are transmitted by the aLP and the vLP, comprises collectively providing DDD operation. In other words, in specific embodiments the coordinated dual chamber operation is DDD operation. The aLP and the vLP collectively providing coordinated dual chamber operation, during which i2i messages are transmitted by the aLP and the vLP (to support the coordinated dual chamber operation), can alternatively comprise collectively providing VDD, DDI, VDI, or DOO operation.

In accordance with certain embodiments, the controller of the aLP is configured to normally disable at least a portion of the receiver of the aLP that enables the aLP to receive one or more i2i messages to thereby conserve power of the power source (e.g., battery) of the aLP compared to if the at least the portion of the receiver of the aLP were enabled. Additionally, the controller of the aLP is configured to from time to time temporarily enable the at least the portion of the receiver of the aLP, that enables the aLP to receive one or more i2i messages, to thereby enable the aLP to monitor for the mode switch type of i2i message that causes the aLP to mode switch from providing the AAI operation to collectively providing with the vLP the coordinated dual chamber operation, during which i2i messages are transmitted by the aLP and the vLP (to support the coordinated dual chamber operation). The controller of the aLP is also configured to cause the aLP to mode switch from providing the AAI operation to collectively providing with the vLP the coordinated dual chamber operation, in response to the aLP receiving and acknowledging the mode switch type of i2i message. In certain such embodiments, the controller of the aLP causes the aLP to abstain from using its transmitter to transmit any i2i messages that are intended to be used to coordinate operation of the vLP with the aLP until the aLP receives a mode switch type of i2i message from the vLP.

In accordance with certain embodiments, the first specified criterion, which the controller of the vLP is configured to determine whether or not is satisfied, comprises the vLP having provided at least a first specified threshold amount of ventricular pacing within a first specified duration.

In accordance with certain embodiments, the controller of the vLP is configured to determine whether or not a second specified criterion is satisfied, after the vLP has mode switched from providing the VVI operation to collectively providing with the aLP the coordinated dual chamber operation. The controller of the vLP is also configured to, during a third period of time that begins in response to the second specified criterion being satisfied, use the transmitter of the vLP to transmit a further mode switch type of i2i message to the aLP, which if successfully received by and acknowledged by the aLP causes the aLP to mode switch from collectively providing with the vLP the coordinated dual chamber operation to the aLP again providing the AAI operation independent of the vLP, and causes the vLP to mode switch from collectively providing with the aLP the coordinated dual chamber operation to the vLP again providing the VVI operation independent of the aLP. In other words, the second specified criterion when satisfied causes a transition from the coordinated dual chamber operation back to the AAI+VVI operation.

In accordance with certain embodiments, the second specified criterion, which the controller of the vLP is configured to determine whether or not is satisfied, comprises the vLP having provided less than a second specified threshold amount of ventricular pacing within a second specified duration.

In accordance with certain embodiments, while the aLP is providing the AAI operation and the vLP is providing the VVI operation, independent of one another, the controller of the aLP is configured to monitor for a third specific criterion that when detected causes the transmitter of the aLP to send an i2i message to the vLP, or to a third IMD that acts as a communication hub for the LPs, instructing the vLP to change its base pacing rate or its base VV interval. In accordance with certain such embodiments, the third specified criterion, which the controller of the aLP is configured to determine whether or not is satisfied, comprises the aLP having detected that the atrial rate exceeds a specified threshold.

This summary is not intended to be a complete description of the embodiments of the present technology. Other features and advantages of the embodiments of the present technology will appear from the following description in which the preferred embodiments have been set forth in detail, in conjunction with the accompanying drawings and claims.

### BRIEF DESCRIPTION OF THE DRAWINGS

Embodiments of the present technology relating to both structure and method of operation may best be understood by referring to the following description and accompanying drawings, in which similar reference characters denote similar elements throughout the several views:
FIG. 1 illustrates a system formed in accordance with certain embodiments herein as implanted in a heart.
FIG. 2 is a block diagram of a single LP in accordance with certain embodiments herein.
FIG. 3 illustrates an LP in accordance with certain embodiments herein.
FIG. 4 is a timing diagram demonstrating one embodiment of i2i communication for a paced event.
FIG. 5 is a timing diagram demonstrating one embodiment of i2i communication for a sensed event.
FIGS. 6A and 6B are high level flow diagrams that are used to summarize operations performed by the system according to certain embodiments of the present technology.
FIG. 7 shows a block diagram of one embodiment of an LP that is implanted into a patient as part of dual chamber LP system in accordance with certain embodiments herein.

### DETAILED DESCRIPTION

As noted above, in order for a dual chamber LP pacing system to provide for coordinated (aka synchronized) dual chamber operation, an aLP and a vLP may coordinate their activities via beat-to-beat i2i communication. However, such beat-to-beat i2i communication (i.e., i2i communication that occurs each and every cardiac cycle) consumes a substantial amount of power, and thus, reduces the longevity of the aLP and the vLP by depleting their respective power supplies (e.g., batteries) thereof faster than would occur if i2i communication were not used at all, or if i2i communication were used less frequently than every beat.

In a dual chamber LP system configured to collectively provide DDD operation, a potential disadvantage of relying on beat-to-beat i2i communications to coordinate pacing of the RA and RV chambers is that such a system may be designed to withhold atrial pacing when i2i communication from the vLP to the aLP is unsuccessful (for a single beat, or across multiple consecutive cardiac cycles), which has the effect of causing the dual chamber LP system to collectively provide VDD operation, rather than collectively providing the programmed DDD operation. This behavior (of collectively providing VDD operation when the programmed DDD operation is not possible) is a design mitigator to prevent the two LPs from potentially causing symptomatic harm to a patient by pacing in a desynchronized (aka uncoordinated) rhythm. While withholding atrial pacing may be acceptable for some patients, there are other patients for which reliable atrial-based pacing is important, and sometimes imperative. For example, for patients that have sinus node dysfunction (SND), atrial-based pacing is the primary therapeutic support needed. Furthermore, most patients with SND have an intact and fully functional atrioventricular (AV) node, so they are able to natively maintain AV synchrony via normal AV conduction followed by intrinsic ventricular activation, with minimal-to-no need for ventricular pacing support.

Certain embodiments of the present technology relate to a new operating mode for a dual chamber LP system, which new operating mode can be referred to herein as an AAI+VVI (AAI "plus" VVI) operating mode, or more succinctly as AAI+VVI mode or AAI+VVI operation, or even more succinctly as AAI+VVI. The primary use case for utilizing this new AAI+VVI mode is with patients having SND who generally have intact AV node conduction, with potentially a rare-to-occasional need for intermittent ventricular pacing support, e.g., due to transient AV block, but not limited thereto. In this context, when a dual chamber LP system is utilizing the new AAI+VVI operating mode, the primary role of the vLP is to provide backup ventricular safety pacing support, which optimally minimizes ventricular pacing to be only when needed by the patient and when pacemaker-mediated atrioventricular (AV) synchrony is not a clinical necessity. That is, the vLP is generally used to provide backup ventricular safety pacing support when the new the AAI+VVI operating mode is being provided.

In accordance with certain embodiments of the present technology, the AAI+VVI operation is achieved by operating the aLP in AAI mode, and operating the vLP in VVI mode, without requiring that the aLP and the vLP perform beat-to-beat i2i communication (i.e., without requiring that the aLP and the vLP attempt to communicate with one another every cardiac cycle). In other words, the AAI+VVI operating mode is achieved by the aLP providing AAI operation and the vLP providing VVI operation during a same period of time.

When the aLP provides the AAI operation (aka, when the aLP provides AAI mode pacing), the aLP performs atrial pacing in response to an intrinsic atrial event not being detected within a specified AA interval following a previous (e.g., an immediately preceding) paced or intrinsic atrial event, performs atrial sensing (to sense for an intrinsic atrial event prior to the end of the specified AA interval), and inhibits the atrial pacing in response to the intrinsic atrial event being detected within the specified AA interval following the previous paced or intrinsic atrial event.

When the vLP provides the VVI operation (aka, when the vLP provides VVI mode pacing), the vLP performs ventricular pacing in response to an intrinsic ventricular event not being detected within a specified VV interval following a previous (e.g., an immediately preceding) paced or intrinsic ventricular event, performs ventricular sensing (to sense for an intrinsic ventricular event prior to the end of the specified VV interval), and inhibits the ventricular pacing in response to the intrinsic ventricular event being detected within the specified VV interval following the previous paced or intrinsic ventricular event.

In accordance with certain embodiments, the programmable pacing rate for the aLP is restricted to always being greater than the programmable pacing rate for the aLP. This constraint ensures that the aLP is driving overall cardiac electromechanical functionality, and therein mitigates against vLP rate superseding aLP rate, which can result in undesirable retrograde AV node conduction. This constraint is achieved, in accordance with certain embodiments, by programming the AA interval (that is used by the aLP) to be shorter than the VV interval (that is used by the vLP), which has the effect of causing an atrial rate of the atrial pacing performed by the aLP to be faster than a ventricular rate of the ventricular pacing performed by the vLP. In certain such embodiments, the aLP provides for rate responsive pacing, i.e., the AAI operation is provided by AAIR operation, while the vLP provides for non-rate responsive VVI pacing. More specifically, in certain embodiments the ability to utilize an activity (e.g., temperature or motion) sensor for modulating pacing rate is restricted to the aLP, e.g., by enabling rate responsive pacing by the aLP, and not made available to the vLP, e.g., by disabling rate responsive pacing by the vLP. This constraint is consistent with the intended use case, where atrial-based pacing is the primary need and the ventricular function tracks intrinsically via intact AV node conduction, with ventricular pacing only intended for backup safety support when needed. In still other embodiments, each of the aLP and the vLP provides for rate responsive pacing, i.e., the aLP provides AAIR operation and the vLP provides VVIR operation.

When referring to various types of operating schemes (aka pacing modes or operations) herein, three letters are typically used to refer to the type of operation. In other words, a three position pacemaker code is often used, with the following nomenclature followed: the first position refers to the cardiac chamber paced; the second position refers to the cardiac chamber sensed; and the third position refers to the response to a sensed event. In the first and second positions, the letter O means none, the letter A means Atrium, the letter V means Ventricle, and the letter D means Dual (i.e., A and V). In the third position the letter O means none, the letter I means Inhibited, the letter T means Triggered (aka Tracked), and the letter D means Dual (i.e., T + I). The below Table 1 summarizes this pacemaker nomenclature. Where an R is included in a fourth position, that means the pacing that is provided is rate responsive.

**Table 1**

| **Position 1** | **Position 2** | **Position 3** |
|---|---|---|
| (Chamber Paced) | (Chamber Sensed) | (Response to Sensed Event) |
| O = none | O = none | O = none |
| A = Atrium | A = Atrium | I = Inhibited |
| V = Ventricle | V = Ventricle | T = Triggered (aka Tracked) |
| D = Dual (A + V) | D = Dual (A + V) | D = Dual (I + T) |

DDD and DDDR operation, as is known in the art, provides for synchronized dual chamber pacing. More specifically, DDD operation provides for atrial and ventricular pacing, atrial and ventricular sensing, and the ability to both inhibit or trigger pacing following a sensed event. DDDR operation is DDD operation that is rate responsive, i.e., the pacing rate is adjusted based on the patient's activity level, e.g., as detected using a motion sensor and/or a temperature sensor. The term DDD operation, as used herein, also encompasses DDDR operation, unless stated otherwise. Similarly, the term AAI operation, as used herein, also encompasses AAIR operation, unless stated otherwise; and the term VVI operation, as used herein, also encompasses VVIR pacing mode, unless stated otherwise.

While neither the aLP nor the vLP can individually perform DDD operation, the aLP and the vLP can be configured to collectively provide for DDD operation (including DDDR operation) when there is successful beat-to-beat bidirectional i2i communication, i.e., when the vLP successfully receives i2i messages from the aLP, and the aLP successfully receives i2i messages from the vLP. In certain embodiments, when the aLP and the vLP collectively provide for DDD operation, they communication with one another each cardiac cycle, i.e., on a beat-to-beat basis.

However, for various different reasons, there may be periods of time during which bidirectional i2i communication fails, because the vLP fails to successfully receive one or more atrial-to-ventricular (a2v) i2i messages from the aLP, and/or the aLP fails to successfully receive one or more ventricular-to-atrial (v2a) i2i messages from the vLP. Such failures in i2i communication may occur, e.g., due to noise, the relative orientations of the aLP and vLP, and/or the distances between the aLP and the vLP, but not limited thereto.

A dual chamber LP system may have safeguards in place to reduce asynchronous pacing in the event of loss of i2i communication in one or both directions, as outlined in the Table 2 below.

**Table 2**

| **Programmed operational mode** | **Automatic functional safeguard mode** | | |
|---|---|---|---|
| | **a2v comm. loss** | **v2a comm. loss** | **a2v and v2a (i.e., bi-directional) comm. loss** |
| DDD | DDI | VDD | VDI |
| DDI | DDI | VDI | VDI |
| VDD | VDI | VDD | VDI |
| DOO | DOO | VOO | VOO |

As can be appreciated from Table 2 above, in a dual chamber LP system including an aLP and vLP that are configured to collectively provide DDD operation, if v2a communication is lost (i.e., one or more v2a i2i messages transmitted by the vLP are not successfully received by the aLP), then the aLP withholds pacing, and the dual chamber LP system effectively provides VDD operation. If a2v i2i communication is lost (i.e., one or more of the a2v i2i messages transmitted by the aLP are not successfully received by the vLP, then tracking is lost and the dual chamber LP system effectively provides DDI operation. If i2i communication is simultaneously lost in both directions (i.e., v2a i2i messages transmitted by the vLP are not successfully received by the aLP, and a2v i2i messages transmitted by the aLP are not successfully received by the vLP), both mitigations take effect, and the dual chamber LP system effectively provides VDI operation. These transmission receipt safeguards act to guarantee RV pacing while maintaining RA tracking and RA pacing whenever possible. However, when the dual chamber LP system effectively provides for VDD or VDI operation, there is no pacing of the atrium.

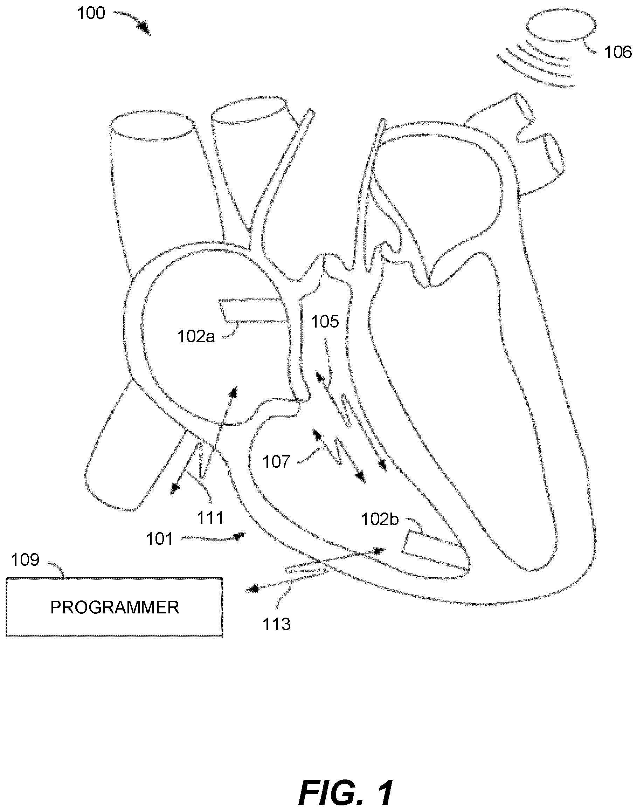

As noted above, while withholding atrial pacing may be acceptable for some patients, there are other patients for which reliable atrial-based pacing is important, and sometimes imperative. For example, for patients that have SND, atrial-based pacing is the primary therapeutic support needed. As noted above, certain embodiments of the present technology relate to a new AAI+VVI operating mode. Additional details of such embodiments are provided herein. However, before providing addition details of the specific embodiments of the present technology mentioned above, an exemplary system in or with which embodiments of the present technology can be used will first be described with reference to FIGS. 1-5. More specifically, FIGS. 1-5 will be used to describe an example dual chamber LP system, which optionally also includes a non-vascular ICD (NV-ICD), such as a subcutaneous-ICD (S-ICD), and a programmer.

FIG. 1 illustrates a system 100 that is configured to be implanted in a heart 101. The system 100 includes LPs 102a and 102b located in different chambers of the heart. LP 102a is located in a right atrium, and thus can also be referred to herein as an atrial LP (aLP). The LP 102b is located in a right ventricle, and thus can also be referred to herein as a ventricular LP (vLP). The aLP 102a and the vLP 102b can be referred to collectively herein as the LPs 102, or individually as an LP 102. The LPs 102a and 102b can communicate with one another to inform one another of various local physiologic activities, such as local intrinsic events, local paced events and the like. The LPs 102a and 102b may be constructed in a similar manner, but operate differently based upon which chamber the LP 102a or 102b is located.

In certain embodiments, LPs 102a and 102b communicate with one another, and/or with an ICD 106, by conductive communication through the same electrodes that are used for sensing and/or delivery of pacing therapy. The LPs 102a and 102b may also be able to use conductive communication to communicate with an external device, e.g., a programmer 109, having electrodes placed on the skin of a patient within with the LPs 102a and 102b are implanted. The LPs 102a and 102b can each alternatively, or additionally, include an antenna that would enable them to communicate with one another, the ICD 106 and/or an external device, using RF communication. Alternatively, or additionally, it is possible that the LPs 102a, 102b utilize another type of communication, such as inductive communication, in which case the LPs 102a, 102b can each include a respective inductive communication coil. While only two LPs are shown in FIG. 1, it is possible that more than two LPs can be implanted in a patient. For example, to provide for bi-ventricular pacing and/or cardiac resynchronization therapy (CRT), in addition to having LPs implanted in the right atrial (RA) chamber and the right ventricular (RV) chamber, a further LP can be implanted in the left ventricular (LV) chamber.

Each LP 102 uses two or more electrodes located within, on, or within a few centimeters of the housing of the pacemaker, for pacing and sensing at the cardiac chamber. Where the LPs 102 communication using conductive communication, the electrodes of the LPs 102 can also be used for bidirectional conductive communication with one another, as well as with the programmer 109, and the ICD 106.

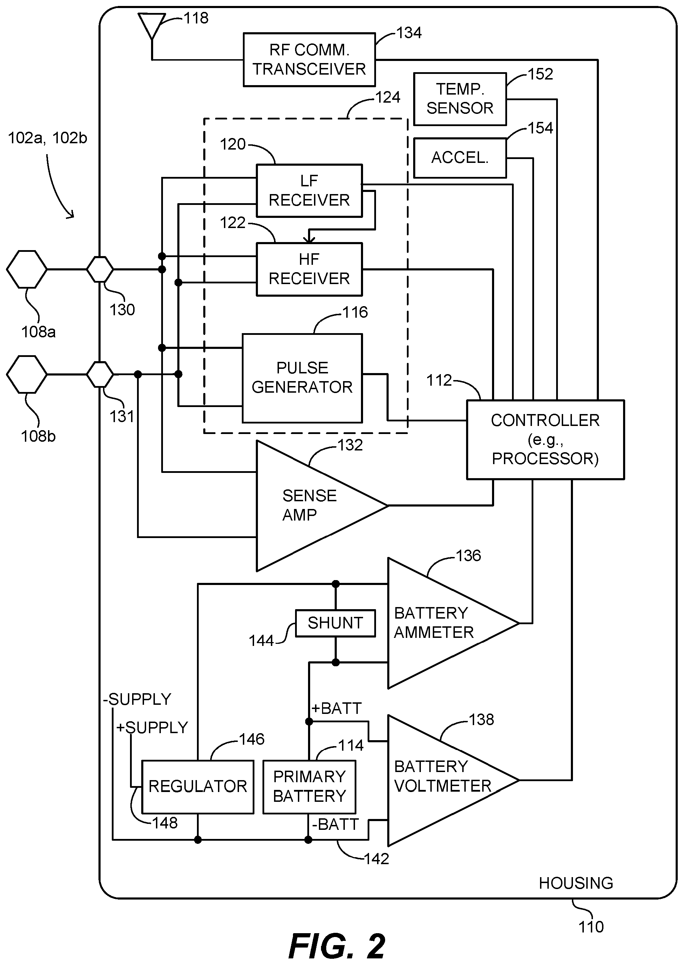

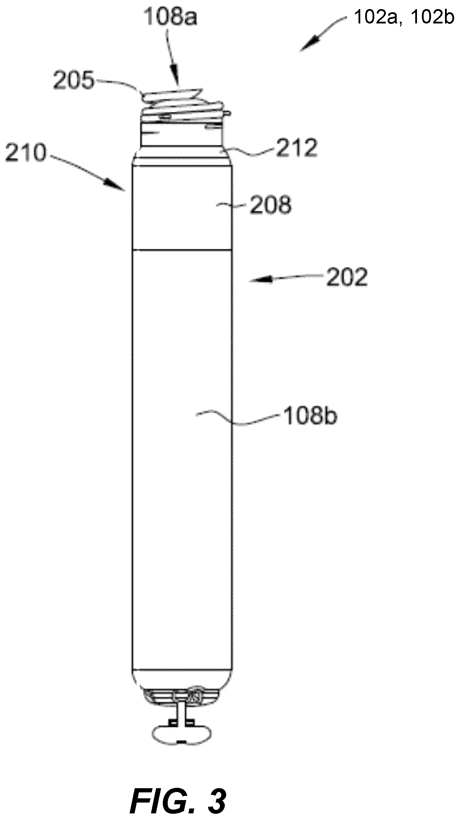

Referring to FIG. 2, a block diagram shows an embodiment for portions of the electronics within LPs 102a, 102b configured to provide conductive communication through the sensing/pacing electrode. One or more of LPs 102a and 102b include at least two leadless electrodes configured for delivering cardiac pacing pulses, sensing evoked and/or natural cardiac electrical signals, and unidirectional or bi-directional conductive communication. In FIG. 2 (and FIG. 3) the two electrodes shown therein are labeled 108a and 108b. Such electrodes can be referred to collectively as the electrodes 108, or individually as an electrode 108. An LP 102, or other type of implantable medical device (IMD), can include more than two electrodes 108, depending upon implementation.

In FIG. 2, each of the LPs 102a, 102b is shown as including first and second receivers 120 and 122 that collectively define separate first and second conductive communication channels 105 and 107 (FIG. 1), (among other things) between LPs 102a and 102b. Although first and second receivers 120 and 122 are depicted, in other embodiments, each LP 102a, 102b may only include the first receiver 120, or more generally may include only a single receiver that is configured to receive conductive communication signals. It is also possible that an LP 102 may include additional receivers other than first and second receivers 120 and 122. As will be described in additional detail below, the pulse generator 116 can function as a transmitter that transmits i2i communication signals using the electrodes 108. In certain embodiments, LPs 102a and 102b may communicate over more than just first and second conductive communication channels 105 and 107. In certain embodiments, LPs 102a and 102b may communicate over one common communication channel 105. More specifically, LPs 102a and 102b can communicate conductively over a common physical channel via the same electrodes 108 that are also used to deliver pacing pulses.

The receivers 120 and 122 can also be referred to, respectively, as a low frequency (LF) receiver 120 and a high frequency (HF) receiver 122, because the receiver 120 is configured to monitor for one or more signals within a relatively low frequency range (e.g., below 100 KHz) and the receiver 122 is configured to monitor for one or more signals within a relatively high frequency range (e.g., above 100 KHz). In certain embodiments, the receiver 120 (and more specifically, at least a portion thereof) is always enabled and monitoring for a wakeup notice, which can simply be a wakeup pulse, within a specific low frequency range (e.g., between 1 KHz and 100 KHz); and the receiver 122 is selectively enabled by the receiver 120. The receiver 120 is configured to consume less power than the receiver 122 when both the first and second receivers are enabled. Accordingly, the receiver 120 can also be referred to as a low power receiver 120, and the receiver 122 can also be referred to as a high power receiver 122. In certain embodiments, while the aLP and the vLP are collectively providing the AAI+VVI operation, the lower power receiver 120 and the high power receiver 120 of the aLP are both normally disable, and the lower power receiver 120 is enabled from time to time to monitor for a mode switch type of i2i message from the vLP. Alternatively, while the aLP and the vLP are collectively providing the AAI+VVI operation, the aLP always powers its low power receiver 120, and only powers its high power receiver 122 in response to receiving a wakeup pulse from the vLP, wherein the wakeup pulse can be part of an i2i message from the vLP that is a mode switch i2i message.

In certain such embodiments, the aLP abstains from using its transmitter to transmit any i2i messages, that are intended to be used to coordinate operation of the vLP with the aLP, until the aLP receives a mode switch type of i2i message.

The low power receiver 120 is incapable of receiving signals within the relatively high frequency range (e.g., above 100 KHz), but consumes significantly less power than the high power receiver 122. This way the low power receiver 120 is capable of always monitoring for a wakeup notice without significantly depleting the battery (e.g., 114) of the LP. In accordance with certain embodiments, the high power receiver 122 is selectively enabled by the low power receiver 120, in response to the low power receiver 120 receiving a wakeup notice, so that the high power receiver 122 can receive the higher frequency signals, and thereby handle higher data throughput needed for effective i2i communication without unnecessarily and rapidly depleting the battery of the LP (which the high power receiver 122 may do if it were always enabled).

Since the receivers 120, 122 are used to receive conductive communication messages, the receivers 120, 122 can also be referred to as conductive communication receivers. In certain embodiments, each of the LPs 102 includes only a single conductive communication receiver.

In accordance with certain embodiments, when one of the LPs 102a and 102b senses an intrinsic event or delivers a paced event, the corresponding LP 102a, 102b transmits an implant event message to the other LP 102a, 102b. For example, when an atrial LP 102a senses/paces an atrial event, the atrial LP 102a transmits an implant event message including an event marker indicative of a nature of the event (e.g., intrinsic/sensed atrial event, paced atrial event). When a ventricular LP 102b senses/paces a ventricular event, the ventricular LP 102b transmits an implant event message including an event marker indicative of a nature of the event (e.g., intrinsic/sensed ventricular event, paced ventricular event). In certain embodiments, each LP 102a, 102b transmits an implant event message to the other LP 102a, 102b preceding the actual pace pulse so that the remote LP can blank its sense inputs (or provide some other type of crosstalk protection) in anticipation of that remote pace pulse (to prevent inappropriate crosstalk sensing). The above describe implant event messages are examples of i2i messages.

The implant event messages may be formatted in various manners. As one example, each event message may include a leading trigger pulse (also referred to as an LP wakeup notice, wakeup pulse or wakeup signal) followed by an event marker. The notice trigger pulse (also referred to as the wakeup notice, wakeup pulse or wakeup signal) is transmitted over a first channel (e.g., with a pulse duration of approximately 10 µs to approximately 1ms and/or within a fundamental frequency range of approximately 1 KHz to approximately 100 KHz). The notice trigger pulse indicates that an event marker is about to be transmitted over a second channel (e.g., within a higher frequency range). The event marker can then be transmitted over the second channel.

The event markers may include data indicative of one or more events (e.g., a sensed intrinsic atrial activation for an atrial located LP, a sensed intrinsic ventricular activation for a ventricular located LP). The event markers may include different markers for intrinsic and paced events. The event markers may also indicate start or end times for timers (e.g., an AV interval, a blanking interval, etc.). Optionally, the implant event message may include a message segment that includes additional/secondary information.

Optionally, the LP (or other IMD) that receives any i2i communication message from another LP (or other IMD) or from an external device may transmit a receive acknowledgement (ACK) indicating that the receiving LP (or other IMD) received the i2i communication message. In certain embodiments, where an IMD expects to receive an i2i communication message within a window, and fails to receive the i2i communication message within the window, the IMD may transmit a failure-to-receive acknowledgement indicating that the receiving IMD failed to receive the i2i communication message. The failure-to-receive acknowledgement message can also be referred to as a negative acknowledgement (NACK) message. An LP can receive a message from another LP that includes an indicator (e.g., an error code) in its payload, or header, that indicates to the LP that the other LP failed to receive an expected message from the LP.

The event messages enable the LPs 102a, 102b to deliver synchronized therapy and additional supportive features (e.g., measurements, etc.). To maintain synchronous therapy, each of the LPs 102a and 102b is made aware (through the event messages) when an event occurs in the chamber containing the other LP 102a, 102b. Some embodiments described herein provide efficient and reliable processes to maintain synchronization between LPs 102a and 102b without maintaining continuous communication between LPs 102a and 102b. In accordance with certain embodiments herein, low power event messages/signaling may be maintained between LPs 102a and 102b synchronously or asynchronously.

For synchronous event signaling, LPs 102a and 102b may maintain synchronization and regularly communicate at a specific interval. Synchronous event signaling allows the transmitter and receivers in each LP 102a,102b to use limited (or minimal) power as each LP 102a, 102b is only powered for a small fraction of the time in connection with transmission and reception. For example, LP 102a, 102b may transmit/receive (Tx/Rx) communication messages in time slots having duration of 10-20µs, where the Tx/Rx time slots occur periodically (e.g., every 10-20ms). Such synchronous event signaling may be used, e.g., when the LPs 102a and 102b collectively provide coordinated dual chamber operation during which i2i messages are transmitted by the aLP and the vLP (to support the coordinated dual chamber operation), but is not limited thereto.

LPs 102a and 102b may lose synchronization, even in a synchronous event signaling scheme. As explained herein, features may be included in LPs 102a and 102b to maintain device synchronization, and when synchronization is lost, LPs 102a and 102b undergo operations to recover synchronization. Also, synchronous event messages/signaling may introduce a delay between transmissions which causes a reaction lag at the receiving LP 102a, 102b. Accordingly, features may be implemented to account for the reaction lag.

During asynchronous event signaling, LPs 102a and 102b do not maintain communication synchronization. During asynchronous event signaling, one or more of receivers 120 and 122 of LPs 102a and 102b may be "always on" (always awake) to search for incoming transmissions. However, maintaining LP receivers 120, 122 in an "always on" (always awake) state presents challenges as the received signal level often is low due to high channel attenuation caused by the patient's anatomy. Further, maintaining the receivers awake will deplete the battery 114 more quickly than may be desirable.

In accordance with certain embodiments, the first receiver 120 may maintain the first channel active (awake) at all times (including when the second channel is inactive (asleep)) in order to listen for messages from a remote LP. The second receiver 122 may be assigned a second activation protocol that is a triggered protocol, in which the second receiver 122 becomes active (awake) in response to detection of trigger events over the first receive channel (e.g., when the incoming signal corresponds to the LP wakeup notice, activating the second channel at the local LP). The terms active, turned on, awake and enabled are used interchangeably herein.

Still referring to FIG. 2, each LP 102a, 102b is shown as including a controller 112 and a pulse generator 116. The controller 112 can include, e.g., a microprocessor (or equivalent control circuitry), RAM and/or ROM memory, logic and timing circuitry, state machine circuitry, and I/O circuitry, but is not limited thereto. The controller 112 can further include, e.g., timing control circuitry to control the timing of the stimulation pulses (e.g., pacing rate, atrio-ventricular (AV) delay, atrial interconduction (AA) delay, or ventricular interconduction (VV) delay, etc.). Such timing control circuitry may also be used for the timing of refractory periods, blanking intervals, noise detection windows, evoked response windows, alert intervals, marker channel timing, and so on. The controller 112 can further include other dedicated circuitry and/or firmware/software components that assist in monitoring various conditions of the patient's heart and managing pacing therapies. For example, the controller 112 can include an arrhythmia detector, which can be similar to the arrhythmia detector 834 discussed below with reference to FIG. 8.

The controller 112 and the pulse generator 116 may be configured to transmit event messages, via the electrodes 108, in a manner that does not inadvertently capture the heart in the chamber where LP 102a, 102b is located, such as when the associated chamber is not in a refractory state. In addition, an LP 102a, 102b that receives an event message may enter an "event refractory" state (or event blanking state) following receipt of the event message. The event refractory/blanking state may be set to extend for a determined period of time after receipt of an event message in order to avoid the receiving LP 102a, 102b from inadvertently sensing another signal as an event message that might otherwise cause retriggering. For example, the receiving LP 102a, 102b may detect a measurement pulse from another LP 102a, 102b or the programmer 109.

Since the conductive communication receivers 120, 122 (or alternatively, just a single conductive communication receiver) and the pulse generator 116 are used to perform conductive communication, these components can be considered parts of a conductive communication transceiver 124. The conductive communication transceiver 124 can alternatively include alternative and/or additional components that enable an LP, or other type of IMD, to transmit and receive conductive communication messages with another IMD and/or with an external device, such as the programmer 109. For an example, while the same pulse generator 116 can be used to produce pacing pulses (for use in pacing a patient's heart) and conductive communication pulses (for use in communicating with another IMD or an external device), it would also be possible that the conductive communication transceiver 124 can include its own dedicated pulse generator. For another example, as noted above, it would also be possible for the conductive communication transceiver 124 to include only a single receiver, rather than both receivers 120, 122.

Still referring to FIG. 2, the LP 102 is also shown as including an antenna 118 that is coupled to a radio frequency (RF) communication transceiver 134, which is configured to transmit and receive RF communication messages using an RF communication protocol, such as a Bluetooth protocol, WiFi protocol, Bluetooth low energy (BLE) protocol, Medical Device Radiocommunications Service (MedRadio) protocol, and/or the like. In certain embodiments, the antenna 118 can be integrated into a fixation mechanism (e.g., 205) of the LP, in which case the antenna can be referred to as a fixation antenna. An example implementation of a fixation antenna is disclosed in U.S. Patent No. 10,583,300, titled "Leadless implantable medical device with fixation antenna member." In other embodiments, the antenna 118 is separate and distinct from the fixation mechanism of the LP 102. The specific type, location and formfactor of the antenna may depend on the specific type and formfactor of the IMD.

The RF communication transceiver 134 consumes more battery power than the conductive communication transceiver 124. More generally, it is more power efficient from an LP 102 (or other type of IMD) to use conductive communication than to use RF communication to communicate with another LP 102 (or other type of IMD). Accordingly, in accordance with certain embodiments of the present technology, the LP 102 (or other type of IMD) is configured to primarily transmit and receive messages using conductive communication, and RF communication is used as a backup or auxiliary type of communication that can be used when conductive communication is deactivated (aka turned off) or is unsuccessful or otherwise deficient, as will be described in additional detail below.

It is possible that the LPs 102a, 102b are only capable of utilizing conductive communication for communicating with one another and/or other devices (such as a programmer 109 and/or ICD 106), in which case the antenna 118 and the RF communication transceiver 134 may be eliminated. It may also be the case that the LPs 102a, 102b are only configured to communicate using RF communication, and to not utilize conductive communication, in which case certain circuitry may be eliminated, such as the receivers 120, 122. Alternatively, or additionally, it is possible that the LPs 102a, 102b utilize another type of communication, such as inductive communication, in which case the LPs 102a, 102b can each include a respective inductive communication coil.

In accordance with certain embodiments herein, the programmer 109 may communicate over a programmer-to-LP channel, with LP 102a, 102b utilizing the same communication scheme. The external programmer 109 may listen to the event message transmitted between LP 102a, 102b and synchronize programmer to implant communication such that programmer 109 does not transmit communication messages 17 until after an i2i messaging sequence is completed.

In some embodiments, each individual LP 102 can comprise a hermetic housing 110 configured for placement on or attachment to the inside or outside of a cardiac chamber and at least two leadless electrodes 108 proximal to the housing 110 and configured for bidirectional communication with at least one other device (e.g., an ICD 106) within or outside the body.

FIG. 2 depicts a single LP 102 (e.g., the LP 102a or 102b) and shows the LP's functional elements substantially enclosed in a hermetic housing 110. The LP 102 has at least two electrodes 108 located within, on, or near the housing 110, for delivering pacing pulses to and sensing electrical activity from the muscle of the cardiac chamber, and for bidirectional communication with at least one other device within or outside the body. Hermetic feedthroughs 130, 131 conduct electrode signals through the housing 110. The housing 110 contains a primary battery 114 to supply power for pacing, sensing, and communication. The housing 110 also contains circuits 132 for sensing cardiac activity from the electrodes 108, receivers 120, 122 for receiving information from at least one other device via the electrodes 108, and the pulse generator 116 for generating pacing pulses for delivery via the electrodes 108 and also for transmitting information to at least one other device via the electrodes 108. The housing 110 can further contain circuits for monitoring device health, for example a battery current monitor 136 and a battery voltage monitor 138, and can contain circuits for controlling operations in a predetermined manner.

The electrodes 108 can be configured to communicate bidirectionally among the multiple leadless cardiac pacemakers and/or the implanted ICD 106 to coordinate pacing pulse delivery and optionally other therapeutic or diagnostic features using messages that identify an event at an individual pacemaker originating the message and a pacemaker receiving the message react as directed by the message depending on the origin of the message. An LP 102a, 102b that receives the event message reacts as directed by the event message depending on the message origin or location. In some embodiments or conditions, the two or more leadless electrodes 108 can be configured to communicate bidirectionally among the one or more leadless cardiac pacemakers 102 and/or the ICD 106 and transmit data including designated codes for events detected or created by an individual pacemaker. Individual pacemakers can be configured to issue a unique code corresponding to an event type and a location of the sending pacemaker.

In some embodiments, an individual LP 102a, 102b can be configured to deliver a pacing pulse with an event message encoded therein, with a code assigned according to pacemaker location and configured to transmit a message to one or more other leadless cardiac pacemakers via the event message coded pacing pulse. The pacemaker or pacemakers receiving the message are adapted to respond to the message in a predetermined manner depending on type and location of the event.

Moreover, information communicated on the incoming channel can also include an event message from another leadless cardiac pacemaker signifying that the other leadless cardiac pacemaker has sensed a heartbeat or has delivered a pacing pulse, and identifies the location of the other pacemaker. For example, LP 102b may receive and relay an event message from LP 102a to the programmer. Similarly, information communicated on the outgoing channel can also include a message to another leadless cardiac pacemaker or pacemakers, or to the ICD, that the sending leadless cardiac pacemaker has sensed a heartbeat or has delivered a pacing pulse at the location of the sending pacemaker.

Referring again to FIGS. 1 and 2, the cardiac pacing system 100 may comprise an ICD 106 in addition to one or more LPs 102a, 102b configured for implantation in electrical contact with a cardiac chamber and for performing cardiac rhythm management functions in combination with the implantable ICD 106. The implantable ICD 106 and the one or more LPs 102a, 102b configured for leadless intercommunication by information conduction through body tissue and/or wireless transmission between transmitters and receivers in accordance with the embodiments discussed herein.

In a further embodiment, a cardiac pacing system 100 comprises at least one LP 102a, 102b configured for implantation in electrical contact with a cardiac chamber and configured to perform cardiac pacing functions in combination with the co-implanted ICD 106. Each LP 102 comprise at least two leadless electrodes 108 configured for delivering cardiac pacing pulses, sensing evoked and/or natural cardiac electrical signals, and transmitting information to the co-implanted ICD 106.

As shown in the illustrative embodiments, an LP 102a, 102b can comprise two or more leadless electrodes 108 configured for delivering cardiac pacing pulses, sensing evoked and/or natural cardiac electrical signals, and bidirectionally communicating with the co-implanted ICD 106.

Each LP 102a, 102b can be configured for operation in a respective particular location and to have a respective particular functionality at manufacture and/or by programming by an external programmer. Bidirectional communication among the multiple leadless cardiac pacemakers can be arranged to communicate notification of a sensed heartbeat or delivered pacing pulse event and encoding type and location of the event to another implanted pacemaker or pacemakers. The LP 102a, 102b receiving the communication decode the information and respond depending on location of the receiving pacemaker and predetermined system functionality.

In some embodiments, the LPs 102a and 102b are configured to be implantable in any chamber of the heart, namely either atrium (RA, LA) or either ventricle (RV, LV). Furthermore, for dual-chamber configurations, multiple LPs may be co-implanted (e.g., one in the RA and one in the RV, one in the RV and one in the coronary sinus proximate the LV). Certain pacemaker parameters and functions depend on (or assume) knowledge of the chamber in which the pacemaker is implanted (and thus with which the LP is interacting; e.g., pacing and/or sensing). Some non-limiting examples include sensing sensitivity, an evoked response algorithm, use of AF suppression in a local chamber, blanking & refractory periods, etc. Accordingly, each LP needs to know an identity of the chamber in (or on) which the LP is implanted, and processes may be implemented to automatically identify a local chamber associated with each LP.

Processes for chamber identification may also be applied to subcutaneous pacemakers, ICDs, with leads and the like. A device with one or more implanted leads, identification and/or confirmation of the chamber into which the lead was implanted could be useful in several pertinent scenarios. For example, for a DR or CRT device, automatic identification and confirmation could mitigate against the possibility of the clinician inadvertently placing the V lead into the A port of the implantable medical device, and vice-versa.

Also shown in FIG. 2, the primary battery 114 has positive terminal 140 and negative terminal 142. Current from the positive terminal 140 of primary battery 114 flows through a shunt 144 to a regulator circuit 146 to create a positive voltage supply 148 suitable for powering the remaining circuitry of the pacemaker 102. The shunt 144 enables the battery current monitor 136 to provide the controller 112 with an indication of battery current drain and indirectly of device health. The illustrative power supply can be a primary battery 114.

Still referring to FIG. 2, the LP is shown as including a temperature sensor 152. The temperature sensor can be any one of various different types of well-known temperature sensors, or can be a future developed temperature sensor. For one example, the temperature sensor 152 can be a thermistor, a thermocouple, a resistance thermometer, or a silicon bandgap temperature sensor, but is not limited thereto. Regardless of how the temperature sensor 152 is implemented, it is preferably that the temperature sensed by the sensor is provided to the controller 112 as a digital signal indicative of the blood temperature of the patient within which the LP is implanted. The temperature sensor 152 can be hermetically sealed within the housing 110, but that need not be the case. The temperature sensor 152 can be used in various manners. For example, the temperature sensor 152 can be used to detect an activity level of the patient to adjust a pacing rate, i.e., for use in rate responsive pacing. When a person starts to exercise their core body temperature initially dips, and then after exercising for a prolonged period of time the person's core body temperature will eventually rise. Thereafter, when the person stops exercising their core body temperature will return to its baseline. Accordingly, the controller 112 can be configured to detect an activity level of a patient based on core blood temperature measurements obtained using the temperature sensor 152.

Referring to FIG. 2, the LP is also shown as including an accelerometer 154 which can be hermetically contained within the housing 110. The accelerometer 154 can be any one of various different types of well-known accelerometers, or can be a future developed accelerometer. For one example, the accelerometer 154 can be or include, e.g., a MEMS (micro-electromechanical system) multi-axis accelerometer of the type exploiting capacitive or optical cantilever beam techniques, or a piezoelectric accelerometer that employs the piezoelectric effect of certain materials to measure dynamic changes in mechanical variables. For example, the accelerometer 154 can be used to detect an activity level of the patient to adjust a pacing rate, i.e., for use in rate responsive pacing. It would also be possible to use outputs of both the accelerometer 154 and the temperature sensor 152 to monitor the activity level of a patient. Alternatively, or additionally, a patient's activity level can be monitored based on their heart rate, as detected from an IEGM sensed using the electrodes 108, and/or sensed using a plethysmography signal obtained using a plethysmography sensor (not shown) or a heart sound sensor (not shown), but not limited thereto. One or more signals produced and output by the accelerometer 154 may be analyzed with respect to frequency content, energy, duration, amplitude and/or other characteristics. Such signals may or may not be amplified and/or filtered prior to being analyzed. For example, filtering may be performed using lowpass, highpass and/or bandpass filters. The signals output by the accelerometer 154 can be analog signals, which can be analyzed in the analog domain, or can be converted to digital signals (by an analog-to-digital converter) and analyzed in the digital domain. Alternatively, the signals output by the accelerometer 154 can already be in the digital domain. The one or more signals output by the accelerometer 154 can be analyzed by the controller 112 and/or other circuitry. In certain embodiments, the accelerometer 154 is packaged along with an integrated circuit (IC) that is designed to analyze the signal(s) it generates. In such embodiments, one or more outputs of the packaged sensor/IC can be an indication of acceleration along one or more axes. In other embodiments, the accelerometer 154 can be packaged along with an IC that performs signal conditioning (e.g., amplification and/or filtering), performs analog-to-digital conversions, and stores digital data (indicative of the sensor output) in memory (e.g., RAM, which may or may not be within the same package). In such embodiments, the controller 112 or other circuitry can read the digital data from the memory and analyze the digital data. In accordance with certain embodiments of the present technology, described in additional detail below, a sensor signal produced by the accelerometer 154 of an LP implanted in or on a cardiac chamber can be used to detect mechanical cardiac activity associated with another cardiac chamber.

In various embodiments, LP 102a, 102b can manage power consumption to draw limited power from the battery, thereby reducing device volume. Each circuit in the system can be designed to avoid large peak currents. For example, cardiac pacing can be achieved by discharging a tank capacitor (not shown) across the pacing electrodes. Recharging of the tank capacitor is typically controlled by a charge pump circuit. In a particular embodiment, the charge pump circuit is throttled to recharge the tank capacitor at constant power from the battery.

In some embodiments, the controller 112 in an LP 102 can access signals on the electrodes 108 and can examine output pulse duration from another LP 102 for usage as a signature for determining triggering information validity and, for a signature arriving within predetermined limits, activating delivery of a pacing pulse following a predetermined delay of zero or more milliseconds. The predetermined delay can be preset at manufacture, programmed via an external programmer, or determined by adaptive monitoring to facilitate recognition of the triggering signal and discriminating the triggering signal from noise. In some embodiments or in some conditions, the controller 112 can examine output pulse waveform from another leadless cardiac pacemaker for usage as a signature for determining triggering information validity and, for a signature arriving within predetermined limits, activating delivery of a pacing pulse following a predetermined delay of zero or more milliseconds.

FIG. 3 shows an example form factor of the LPs 102a, 102b. Each LP can include a hermetic housing 202 (e.g., 110 in FIG. 2) with electrodes 108a and 108b disposed thereon. As shown, electrode 108a can be separated from but surrounded partially by a fixation mechanism 205, and the electrode 108b can be disposed on the housing 202. The fixation mechanism 205 can be a fixation helix, a plurality of hooks, barbs, or other attaching features configured to attach the pacemaker to tissue, such as heart tissue. As noted above, an antenna (e.g., 118) can be at least partially implanted by or as part of the fixation mechanism. The electrodes 108a and 108b are examples of the electrodes 108 shown in and discussed above with reference to FIG. 2.

The housing can also include an electronics compartment 210 within the housing that contains the electronic components necessary for operation of the pacemaker, including, e.g., a pulse generator, transceiver, a battery, and a processor for operation. The hermetic housing 202 can be adapted to be implanted on or in a human heart, and can be cylindrically shaped, rectangular, spherical, or any other appropriate shapes, for example.

The housing can comprise a conductive, biocompatible, inert, and anodically safe material such as titanium, 316L stainless steel, or other similar materials. The housing can further comprise an insulator disposed on the conductive material to separate electrodes 108a and 108b. The insulator can be an insulative coating on a portion of the housing between the electrodes, and can comprise materials such as silicone, polyurethane, parylene, or another biocompatible electrical insulator commonly used for implantable medical devices. In the embodiment of FIG. 2, a single insulator 208 is disposed along the portion of the housing between electrodes 108a and 108b. In some embodiments, the housing itself can comprise an insulator instead of a conductor, such as an alumina ceramic or other similar materials, and the electrodes can be disposed upon the housing.

As shown in FIG. 3, the pacemaker can further include a header assembly 212 to isolate 108a and 108b. The header assembly 212 can be made from PEEK, tecothane or another biocompatible plastic, and can contain a ceramic to metal feedthrough, a glass to metal feedthrough, or other appropriate feedthrough insulator as known in the art.

The electrodes 108a and 108b can comprise pace/sense electrodes, or return electrodes. A low-polarization coating can be applied to the electrodes, such as sintered platinum, platinum-iridium, iridium, iridium-oxide, titanium-nitride, carbon, or other materials commonly used to reduce polarization effects, for example. In FIG. 2, electrode 108a can be a pace/sense electrode and electrode 108b can be a return electrode. The electrode 108b can be a portion of the conductive housing 202 that does not include an insulator 208.

Several techniques and structures can be used for attaching the housing 202 to the interior or exterior wall of the heart. A helical fixation mechanism 205, can enable insertion of the device endocardially or epicardially through a guiding catheter. A torqueable catheter can be used to rotate the housing and force the fixation device into heart tissue, thus affixing the fixation device (and also the electrode 108a in FIG. 2) into contact with stimulable tissue. Electrode 108b can serve as an indifferent electrode for sensing and pacing. The fixation mechanism may be coated partially or in full for electrical insulation, and a steroid-eluting matrix may be included on or near the device to minimize fibrotic reaction, as is known in conventional pacing electrode-leads.

### Implant-to-Implant Event Messaging

LPs 102a and 102b can utilize implant-to-implant (i2i) communication through event messages to coordinate operation with one another in various manners. The terms i2i event messages and i2i event markers are used interchangeably herein to refer to event related messages and IMD/IMD operation related messages transmitted from an implanted device and directed to another implanted device (although external devices, e.g., a programmer, may also receive i2i event messages). In certain embodiments, LP 102a and LP 102b operate as two independent leadless pacers maintaining beat-to-beat dual-chamber functionality via a "Master/Slave" operational configuration. For descriptive purposes, the ventricular LP 102b shall often be referred to as "vLP" and the atrial LP 102a shall often be referred to as "aLP," as was noted above. The LP 102 that is designated as the master device (e.g., vLP) may implement all or most dual-chamber diagnostic and therapy determination algorithms. For purposes of the following illustration, it is assumed that the vLP is a "master" device, while the aLP is a "slave" device. Alternatively, the aLP may be designated as the master device, while the vLP may be designated as the slave device. The master device orchestrates most or all decision-making and timing determinations (including, for example, rate-response changes).

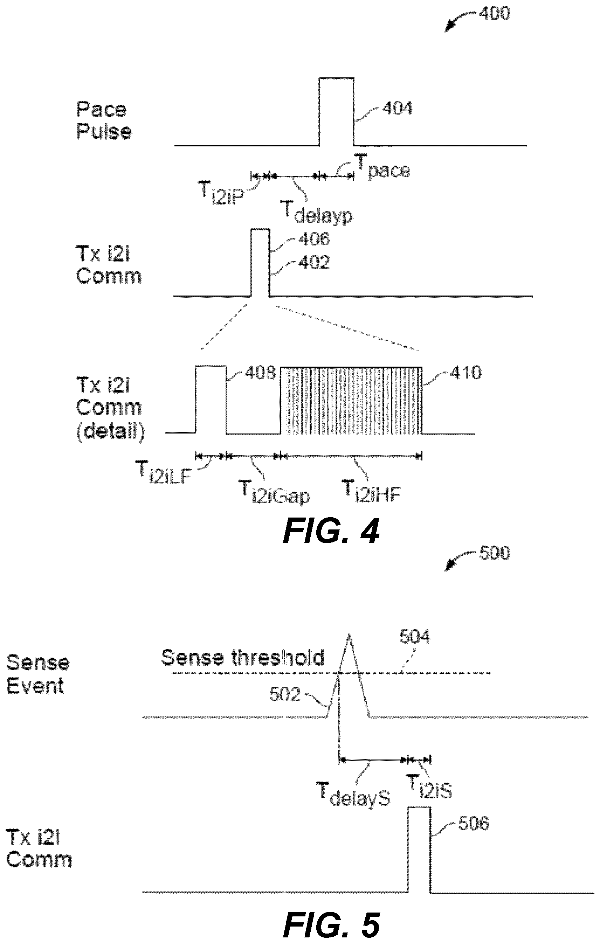

FIG. 4 is a timing diagram 400 demonstrating one example of an i2i conductive communication for a paced event. The i2i conductive communication may be transmitted, for example, from LP 102a to LP 102b. As shown in FIG. 4, in this embodiment, an i2i transmission 402 is sent prior to delivery of a pace pulse 404 by the transmitting LP (e.g., LP 102a). This enables the receiving LP (e.g., LP 102b) to prepare for the remote delivery of the pace pulse. The i2i transmission 402 includes an envelope 406 that may include one or more individual pulses. For example, in this embodiment, envelope 406 includes a low frequency pulse 408 followed by a high frequency pulse train 410. Low frequency pulse 408 lasts for a period T_{i2iLF}, and high frequency pulse train 410 lasts for a period T_{i2iHF}. The end of the low frequency pulse 408 and the beginning of the high frequency pulse train 410 are separated by a gap period, T_{i2iGap}. In alternative embodiments, rather than transmitting the envelope 406 prior to the pacing pulse 404, the envelope 406 can be transmitted during a refractory period that follows the delivery of the pacing pulse.

As shown in FIG. 4, the i2i transmission 402 lasts for a period Ti2iP, and pace pulse 404 lasts for a period Tpace. The end of i2i transmission 402 and the beginning of pace pulse 404 are separated by a delay period, TdelayP. The delay period may be, for example, between approximately 0.0 and 10.0 milliseconds (ms), particularly between approximately 0.1 ms and 2.0 ms, and more particularly approximately 1.0 ms. The term approximately, as used herein, means +/- 10% of a specified value.

FIG. 5 is a timing diagram 500 demonstrating one example of an i2i communication for a sensed event. The i2i communication may be transmitted, for example, from LP 102a to LP 102b. As shown in FIG. 5, in this embodiment, the transmitting LP (e.g., LP 102a detects the sensed event when a sensed intrinsic activation 502 crosses a sense threshold 504. A predetermined delay period, T_{delayS}, after the detection, the transmitting LP transmits an i2i transmission 506 that lasts a predetermined period T_{i2iS}. The delay period may be, for example, between approximately 0.0 and 10.0 milliseconds (ms), particularly between approximately 0.1 ms and 2.0 ms, and more particularly approximately 1.0 ms.

As with i2i transmission 402, i2i transmission 506 may include an envelope that may include one or more individual pulses. For example, similar to envelope 406, the envelope of i2i transmission 506 may include a low frequency pulse followed by a high frequency pulse train. In certain embodiments, the i2i transmission 506 is transmitted during a refractory period that follows the sensed event.

Optionally, wherein the first LP is located in an atrium and the second LP is located in a ventricle, the first LP produces an AS/AP event marker to indicate that an atrial sensed (AS) event or atrial paced (AP) event has occurred or will occur in the immediate future. For example, the AS and AP event markers may be transmitted following the corresponding AS or AP event. Alternatively, the first LP may transmit the AP event marker slightly prior to delivering an atrial pacing pulse. Alternatively, wherein the first LP is located in an atrium and the second LP is located in a ventricle, the second LP initiates an atrioventricular (AV) interval after receiving an AS or AP event marker from the first LP; and initiates a post atrial ventricular blanking (PAVB) interval after receiving an AP event marker from the first LP.

Optionally, the first and second LPs may operate in a "pure" master/slave relation, where the master LP delivers "command" markers in addition to or in place of "event" markers. A command marker directs the slave LP to perform an action such as to deliver a pacing pulse and the like. For example, when a slave LP is located in an atrium and a master LP is located in a ventricle, in a pure master/slave relation, the slave LP delivers an immediate pacing pulse to the atrium when receiving an AP command marker from the master LP.

In accordance with some embodiments, communication and synchronization between the aLP and vLP is implemented via conducted communication of markers/commands in the event messages (per i2i communication protocol). As explained above, conducted communication represents event messages transmitted from the sensing/pacing electrodes at frequencies outside the RF (e.g., Wi-Fi or BLE) frequency range. Alternatively, the event messages may be conveyed over communication channels operating in the RF frequency range. The figures and corresponding description below illustrate non-limiting examples of markers that may be transmitted in event messages. The figures and corresponding description below also include the description of the markers and examples of results that occur in the LP that receives the event message. Table 3 represents example event markers sent from the aLP to the vLP, while Table 4 represents example event markers sent from the vLP to the aLP. In the master/slave configuration, AS event markers are sent from the aLP each time that an atrial event is sensed outside of the post ventricular atrial blanking (PVAB) interval or some other alternatively-defined atrial blanking period. The AP event markers are sent from the aLP each time that the aLP delivers a pacing pulse in the atrium. The aLP may restrict transmission of AS markers, whereby the aLP transmits AS event markers when atrial events are sensed both outside of the PVAB interval and outside the post ventricular atrial refractory period (PVARP) or some other alternatively-defined atrial refractory period. Alternatively, the aLP may not restrict transmission of AS event markers based on the PVARP, but instead transmit the AS event marker every time an atrial event is sensed.

**Table 3**

| **"A2V" Markers / Commands (i.e., from aLP to vLP)** | | |
|---|---|---|
| *Marker* | *Description* | *Result in vLP* |
| AS | Notification of a sensed event in atrium (if not in PVAB or PVARP) | • Initiate AV interval (if not in PVAB or PVARP) |
| AP | Notification of a paced event in atrium | • Initiate PAVB |
| | | • Initiate AV interval (if not in PVARP) |

As shown in Table 3, when an aLP transmits an event message that includes an AS event marker (indicating that the aLP sensed an intrinsic atrial event), the vLP initiates an AV interval timer. If the aLP transmits an AS event marker for all sensed events, then the vLP would preferably first determine that a PVAB or PVARP interval is not active before initiating an AV interval timer. If however the aLP transmits an AS event marker only when an intrinsic signal is sensed outside of a PVAB or PVARP interval, then the vLP could initiate the AV interval timer upon receiving an AS event marker without first checking the PVAB or PVARP status. When the aLP transmits an AP event marker (indicating that the aLP delivered or is about to deliver a pace pulse to the atrium), the vLP initiates a PVAB timer and an AV interval time, provided that a PVARP interval is not active. The vLP may also blank its sense amplifiers to prevent possible crosstalk sensing of the remote pace pulse delivered by the aLP.

**Table 4**

| **"V2A" Markers / Commands (*i.e*., from vLP to aLP)** | | |
|---|---|---|
| *Marker* | *Description* | *Result in aLP* |
| VS | Notification of a sensed event in ventricle | • Initiate PVARP |
| VP | Notification of a paced event in ventricle | • Initiate PVAB |
| | | • Initiate PVARP |
| AP | Command to deliver immediate pace pulse in atrium | • Deliver immediate pace pulse to atrium |

As shown in Table 4, when the vLP senses a ventricular event, the vLP transmits an event message including a VS event marker, in response to which the aLP may initiate a PVARP interval timer. When the vLP delivers or is about to deliver a pace pulse in the ventricle, the vLP transmits VP event marker. When the aLP receives the VP event marker, the aLP initiates the PVAB interval timer and also the PVARP interval timer. The aLP may also blank its sense amplifiers to prevent possible crosstalk sensing of the remote pace pulse delivered by the vLP. The vLP may also transmit an event message containing an AP command marker to command the aLP to deliver an immediate pacing pulse in the atrium upon receipt of the command without delay.

The foregoing event markers are examples of a subset of markers that may be used to enable the aLP and vLP to maintain full dual chamber functionality. In one embodiment, the vLP may perform all dual-chamber algorithms, while the aLP may perform atrial-based hardware-related functions, such as PVAB, implemented locally within the aLP. In this embodiment, the aLP is effectively treated as a remote 'wireless' atrial pace/sense electrode. In another embodiment, the vLP may perform most but not all dual-chamber algorithms, while the aLP may perform a subset of diagnostic and therapeutic algorithms. In an alternative embodiment, vLP and aLP may equally perform diagnostic and therapeutic algorithms. In certain embodiments, decision responsibilities may be partitioned separately to one of the aLP or vLP. In other embodiments, decision responsibilities may involve joint inputs and responsibilities.

Messages that are transmitted between LPs (e.g., the aLP and the vLP) can be referred to herein generally as i2i messages, since they are implant-to-implant messages. As noted above, such messages can include event markers that enable one LP to inform the other LP of a paced event or a sensed event. For example, in certain embodiments, whenever the aLP 102a senses an atrial event or paces the right atrium, the aLP will transmit an i2i message to the vLP 102b to inform the vLP of the sensed or paced event in the atrium. In response to receiving such an i2i message, the vLP 102b may start one or more timers that enable the vLP to sense or pace in the right ventricle. Similarly, the vLP may transmit an i2i message to the aLP 102a whenever the vLP senses a ventricular event or paces the right ventricle.

The i2i messages that are sent between LPs may be relatively short messages that simply allow a first LP to inform a second LP of an event that was sensed by the first LP or caused (paced) by the first LP, and vice versa. Such i2i messages can be referred to herein as event marker i2i messages, or more succinctly as event i2i messages. The i2i messages that are sent between LPs, in certain instances, can be extended i2i messages that include (in addition to an event marker) an extension. In certain embodiments, an extended i2i message includes an event marker (e.g., 9 bits), followed by an extension indicator (e.g., 2 bits), followed by an extended message payload portion (e.g., 17 bits), followed by a cyclic redundancy check (CRC) code (e.g., 6 bits) or some other type of error detection and correction code. Other variations are also possible.

### AAI+VVI operation

As explained above, certain embodiments of the present technology relate to a new AAI+VVI operating mode for use by a dual chamber LP system. As also explained above, a primary use case for utilizing this new AAI+VVI operation is with patients having SND who generally have intact AV node conduction, with potentially a rare-to-occasional need for intermittent ventricular pacing support, e.g., due to transient AV block, but not limited thereto.

The AAI+VVI operation is achieved by operating the aLP in AAI mode, and operating the vLP in VVI mode, without requiring that the aLP and the vLP perform i2i communication (i.e., without requiring that the aLP and the vLP attempt to communicate with one another every cardiac cycle, every other cardiac cycle, or even less frequently). In other words, the AAI+VVI operating mode is achieved by the aLP providing AAI operation and the vLP independently providing VVI operation during a same period of time.

When the aLP provides the AAI operation, the aLP performs atrial pacing in response to an intrinsic atrial event not being detected within a specified AA interval following a previous (e.g., an immediately preceding) paced or intrinsic atrial event, performs atrial sensing (to sense for an intrinsic atrial event prior to the end of the specified AA interval), and inhibits the atrial pacing in response to the intrinsic atrial event being detected within the specified AA interval following the previous paced or intrinsic atrial event.

When the vLP provides the VVI operation, the vLP performs ventricular pacing in response to an intrinsic ventricular event not being detected within a specified VV interval following a previous (e.g., an immediately preceding) paced or intrinsic ventricular event, performs ventricular sensing (to sense for an intrinsic ventricular event prior to the end of the specified VV interval), and inhibits the ventricular pacing in response to the intrinsic ventricular event being detected within the specified VV interval following the previous paced or intrinsic ventricular event.

In accordance with certain embodiments, the programmable pacing rate for the aLP is restricted to always being greater than the programmable pacing rate for the vLP. This constraint ensures that the aLP is driving overall cardiac electromechanical functionality, and therein mitigates against the vLP rate superseding the aLP rate, which can result in undesirable retrograde AV node conduction. This constraint is achieved, in accordance with certain embodiments, by programming the AA interval (that is used by the aLP) to be shorter than the VV interval (that is used by the vLP), which has the effect of causing an atrial rate of the atrial pacing performed by the aLP to be faster than a ventricular rate of the ventricular pacing performed by the vLP. Additionally or alternatively, in accordance with certain embodiments, the programmable pacing rate for the vLP is restricted to always being less than the programmable pacing rate for the aLP. This constraint similarly ensures that the aLP is driving overall cardiac electromechanical functionality, and therein mitigates against the vLP rate superseding the aLP rate, which can result in undesirable retrograde AV node conduction. This constraint is achieved, in accordance with certain embodiments, by programming the VV interval (that is used by the vLP) to be longer than the AA interval (that is used by the aLP), which has the effect of causing a ventricular rate of the ventricular pacing performed by the vLP to be slower than an atrial rate of the atrial pacing performed by the aLP.

In accordance certain embodiments, the aLP provides for rate responsive pacing, i.e., the AAI operation is provided by AAIR operation, while the vLP provides for non-rate responsive VVI pacing. More specifically, the aLP can utilize a temperature sensor (e.g., 152) thereof and/or a motion sensor (e.g., accelerometer 154) thereof to detect patient activity, and modulate its pacing rate based on the detected patient activity. In certain such embodiments, while the aLP is providing rate responsive AAI operation, i.e., AAIR operation, the vLP provides its VVI operation utilizing a constant programmed ventricular rate. In other words, the VVI operation provided by the vLP is not rate responsive. This can be because the vLP disables its temperature sensor (e.g., 152) and/or its motion sensor (e.g., accelerometer 154), ignores the outputs thereof, or turns off its rate responsive feature. Such one or more constraints is/are consistent with the intended use case, where atrial-based pacing by the aLP is the primary need and the ventricular function provided by the vLP tracks intrinsically via intact AV node conduction, with ventricular pacing by the vLP only intended for backup safety support when needed. In other embodiments, while the aLP is providing rate responsive AAI operation, i.e., AAIR operation, the vLP is providing rate responsive VVI operation, i.e., VVIR operation, which can be referred to more specifically as AAIR+VVIR operation. In certain embodiments, when the dual chamber LP system is providing AAIR+VVIR operation, a sensitivity of the temperature sensor and/or motion sensor of the vLP is less than a sensitivity of the temperature sensor and/or motion sensor of the aLP, so that an increase in the atrial pacing rate provided by aLP is greater than a corresponding increase in the ventricular pacing rate provided by vLP. Such a configuration keeps the AA interval shorter than the VV interval to thereby cause an atrial rate of the atrial pacing performed by the aLP to remain faster than a ventricular rate of the ventricular pacing performed by the vLP.

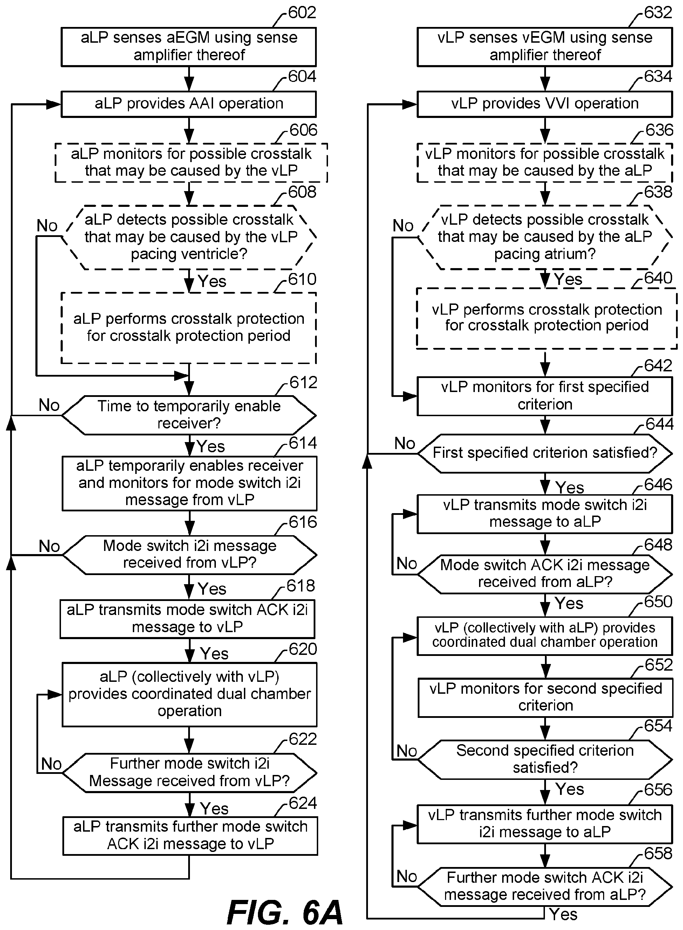

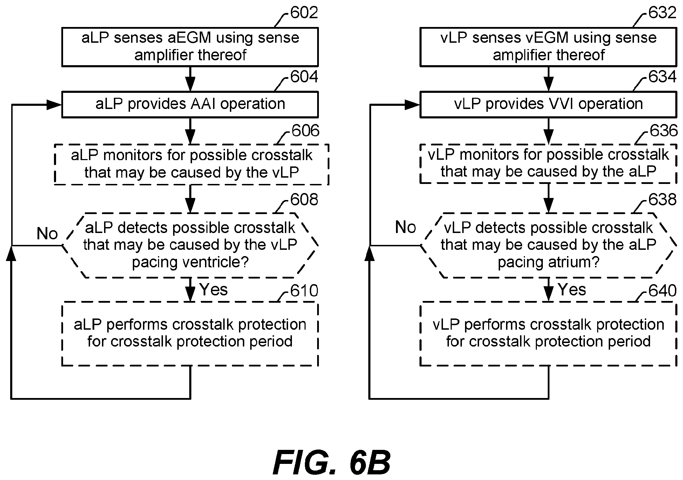

Additional details of the AAI+VVI operation according to certain embodiments of the present technology are described below with reference to the high level flow diagrams of FIGS. 6A and 6B. In FIGS. 6A and 6B, steps or operations that are performed by the aLP (e.g., 102a) are shown at the left, and steps or operations that are performed by the vLP (e.g., 102b) are shown at the right. When describing FIG. 6A and other embodiments, it is often assumed that the aLP and the vLP are capable of directly communicating with one another using i2i messages sent from the aLP to the vLP, and using i2i messages sent from the vLP to the aLP. However, it is noted that a third implantable medical device (IMD) can alternatively act as a communication hub for the aLP and the vLP, in which case the aLP can send an i2i message to the third IMD and the third IMD can send the i2i message to the vLP, and the vLP can send a further i2i message to the third IMD and the third IMD can send the further i2i message to the aLP. Such a third IMD can be, e.g., the ICD 106, an insertable cardiac monitor (ICM), or an IMD that is solely used for supporting communication between other IMDs, but is not limited thereto.

Referring to FIG. 6A, step 602 involves the aLP using its sense amplifier (e.g., 132) to sense an atrial electrogram (aEGM), which the aLP (and more specifically, the controller thereof) uses to detect intrinsic atrial events, such as P-waves. Step 604 involves the aLP providing AAI operation, during which the aLP performs atrial pacing when an intrinsic atrial event is not detected within a specified AA interval following a previous paced or intrinsic atrial event, performs atrial sensing (e.g., to sense for an intrinsic atrial event within the aEGM), and inhibits the atrial pacing when the intrinsic atrial event is detected within the specified AA interval following the previous (e.g., immediately preceding) paced or intrinsic atrial event. It is noted that the AAI operation provided at step 604 can be rate responsive, i.e., can be AAIR operation, depending upon the specific implementation. Alternatively, it is also possible that the AAI operation provided at step 604 is not rate responsive.

Still referring to FIG. 6A, step 632 involves the vLP using its sense amplifier (e.g., 132) to sense a ventricular electrogram (vEGM), which the vLP (and more specifically, the controller thereof) uses to detect intrinsic ventricular events, such as R-waves. Step 634 involves the vLP providing VVI operation, during which the vLP performs ventricular pacing when an intrinsic ventricular event is not detected within a specified VV interval following a previous paced or intrinsic ventricular event, performs ventricular sensing (e.g., to sense for an intrinsic ventricular event within the vEGM), and inhibits the ventricular pacing when the intrinsic ventricular event is detected within the specified VV interval following the previous (e.g., immediately preceding) paced or intrinsic ventricular event.

In accordance with certain embodiments, steps 602 and 604 are performed by the aLP during a same period of time that steps 632 and 634 are performed by the vLP. In certain such embodiments, in order to conserve power and thereby increase longevity of the aLP and the vLP, the aLP abstains from transmitting any i2i messages that are intended to be used to coordinate operation of the vLP with the aLP while the vLP is providing the VVI operation, and the vLP abstains from transmitting any i2i messages that are intended to be used to coordinate operation of the aLP with the vLP while the aLP is providing the AAI operation, while steps 604 and 634 are being performed. In other words, AAI+VVI operation is achieved without requiring any i2i messages being sent from the aLP to the vLP (directly, or via a third IMD that acts as a communication hub), and vice versa.

If the aLP and the vLP were communicating with one another using i2i messages, then the vLP may inform the aLP via an i2i message of a pacing pulse that is about to be delivered by the vLP, in response to which the aLP could blank its sense amplifier (e.g., 132) so that the aLP doesn't mistakenly detect a paced ventricular event as an intrinsic atrial event. However, this is not possible when the aLP and the vLP abstain from sending i2i messages to one another, which increases the probability that the aLP could mistakenly detect a paced ventricular event as an intrinsic atrial event, which in turn would cause the aLP to inhibit atrial pacing for that cardiac cycle, which is undesirable. To reduce the probability of the aLP mistakenly detecting a paced ventricular event as an intrinsic atrial event, at step 606 the aLP monitors for possible crosstalk that may be caused by the vLP performing ventricular pacing. According, in accordance with certain embodiments the aLP is configured to monitor for possible crosstalk that may be caused by the vLP performing ventricular pacing. Example additional details of how such embodiments can be implemented are described below.

Such possible crosstalk monitoring at step 606 can be performed, for example, using one of the techniques described in U.S. Patent No. 10,182,765, titled "Systems and Methods for Classifying Signals of Interest in a Cardiac Rhythm Management Device." For example, referring to FIG. 2, assume the sense amplifier 132 (in FIG. 2) of an LP is used to pass signals within a first frequency passband that contains intrinsic cardiac activity (e.g., P-waves and R-waves), then the sense amplifier 132 can also be referred to as an intrinsic activity sense amplifier 132 or an intrinsic activation sensing circuit, or the like. In order to monitor for possible crosstalk at step 606, the aLP (e.g., 102a) can also include a separate crosstalk sensing circuit that has a passband that is shifted to a significantly higher frequency than the passband of the intrinsic activity sense amplifier 132, to enable the crosstalk sensing circuity to be more discriminative of the relatively fast rising and falling edges of crosstalk caused by a pacing pulse produced by the vLP (e.g., 102b). Such a crosstalk sensing circuit can be implemented, for example, by the LF receiver 120, or can by another sense amplifier not specifically shown in FIG. 2. Using such sensing circuits, the aLP, and more specifically a controller (e.g., 112) thereof, can classify a sensed signal being evaluated as being possible crosstalk where the sensed signal is passed by the crosstalk sensing circuit. This is just one example of circuits and techniques that the aLP can use to perform possible crosstalk monitoring at step 606.

Referring again to FIG. 6A, at step 608 there is a determination of whether or not the aLP detects possible crosstalk that may be caused by the vLP performing ventricular pacing, and more specifically, the vLP delivering a pacing pulse. If the answer to the determination at step 608 is No, then step 610 is skipped and flow goes to step 612. If the answer to the determination at step 608 is Yes, then flow goes to step 610. At step 610 the aLP initiates providing crosstalk protection for a crosstalk protection period, so as to avoid crosstalk caused by a ventricular pacing pulse from being mistakenly detected as an intrinsic atrial event. Such crosstalk protection can be performed in various different manners. For example, the intrinsic activity sense amplifier 132 can be blanked by shorting its two inputs to one another, or to ground, such that there is a zero voltage differential between the voltages at the differential inputs. Alternatively, or additionally, the output of the intrinsic activity sense amplifier 132 may not be provided to the controller 112 during the crosstalk protection period (e.g., by opening a switch). Alternatively, or additionally, the output of the intrinsic activity sense amplifier 132 may be ignored by the controller 112 during the crosstalk protection period, i.e., the crosstalk protection period may be implemented by firmware and/or software of the controller 112. Instead of performing blanking at step 610, the aLP (and more specifically its controller) can ignore an intrinsic atrial event that is detected during the crosstalk protection period, because it can be assumed that such a detected intrinsic atrial event is actually crosstalk caused by the vLP performing ventricular pacing. In certain embodiments, crosstalk protection can involve, for example, blanking a sense circuit for the period of time, which sense circuit the LP uses to monitor for intrinsic events of the chamber in (or on) which the LP is implanted. Alternatively, or additionally, the crosstalk protection (that is performed in response to detecting possible crosstalk) can involve ignoring any possible intrinsic depolarization detected using the sense circuit of the LP during the period of time, disabling the sense circuit of the LP for the period of time, ignoring any interrupts (produced in response to the sense circuit being used to detect a possible intrinsic depolarization) during the period of time, or disabling generating of interrupts (that may be produced in response to the sense circuit detecting an intrinsic depolarization) during the period of time, but is not limited thereto. Such crosstalk protection may be performed under the assumption that any possible intrinsic depolarization(s) that was/were detected while crosstalk protection is being performed was/were actually due to the crosstalk. Following step 608, flow goes to step 612. The blocks corresponding to steps 606, 608, and 610 in FIG. 6A are shown in dashed lines to show that such steps are optional, and that it is alternatively possible that flow goes directly from step 604 to step 612.

As noted above, in certain embodiments, while steps 604 and 634 are being performed to provide the AAI+VVI operation, there is no i2i communication between the aLP and the vLP. In other words, AAI+VVI operation is achieved without requiring any i2i messages be sent from the aLP to the vLP (directly, or via a third IMD that acts as a communication hub), and vice versa. While i2i communication is not being used by the aLP and the vLP, the aLP can normally disable its receiver(s), e.g., 120 and 122, and temporarily enable one or more of its receiver(s) from time to time, to sense for possible crosstalk and/or to determine whether a mode switch message is received from the vLP. More specifically, at step 612 the aLP determines whether or not it is time to temporarily enable its receiver 120 to monitor for a mode switch type of i2i message from the vLP (or a third IMD that acts as a communication hub). If the answer to the determination at step 612 is No, then flow returns to step 604 and the aLP continues to provide AAI operation. If the answer to the determination at step 612 is Yes, then flow goes to step 614 and the aLP temporarily enables its receiver 120 to monitor for a mode switch type of i2i message from the vLP (or a third IMD).

For example, the aLP can at step 614 temporarily enable its receiver 120, once per period of time (e.g., once every 10 seconds, once every minute, once every 5 minutes, or once every 10 minutes, etc.) for a specified duration (e.g., 1 second, 2 seconds, 5 seconds, or 10 seconds, etc.) or for a specified number of cardiac cycles (e.g., 1 cardiac cycle, 2 cardiac cycles, 5 cardiac cycles, or 10 cardiac cycles, etc.). Alternatively, the aLP can temporarily enable its receiver 120, once every specified number of cardiac cycles (e.g., 10 cardiac cycles, 30 cardiac cycles, 60 cardiac cycles, or 120 cardiac cycles, etc.) for a specified duration (e.g., 1 second, 2 seconds, 5 seconds, or 10 seconds, etc.) or for a specified number of cardiac cycles (e.g., 1 cardiac cycle, 2 cardiac cycles, 5 cardiac cycles, or 10 cardiac cycles, etc.). It is also possible that the low power receiver 120 of the aLP is always on and selectively temporarily enables the high power receiver 122 in response to the low power receiver 120 receiving a wakeup pulse of an i2i mode switch message received from the vLP. If the low power receiver 120 of the aLP is already enabled to provide crosstalk protection (in accordance with steps 606, 608, and 610), or is always on (aka always enabled) and is in the process of providing crosstalk protection (by performing one of steps 606, 608, or 610), then at step 612 the aLP can be reconfigured to start listening for and processing possible i2i messages. Such reconfiguring of the aLP could be achieved, for example, by updating low-level i2i communication hardware registers to enable the i2i hardware (e.g., the high power receiver 122) to receive i2i payloads, or it could be a logical reconfiguration, but is not limited thereto.

At step 616, there is a determination of whether or not the aLP received a mode switch type i2i message from the vLP during the period of time that the receiver(s), or a portion thereof, of the aLP is/are enabled. If the answer to the determination at step 616 is No, then flow returns to step 604 and the aLP continues to provide AAI operation. If the answer to the determination at step 616 is Yes, then flow goes to step 618. In certain embodiments, the aLP abstains from transmitting any i2i messages that are intended to be used to coordinate operation of the vLP with the aLP until the aLP receives a mode switch type of i2i message from the vLP (or a third IMD).

At step 618 the aLP transmits a mode switch acknowledgement (ACK) type i2i message to the vLP, and then the aLP changes its mode of operation from providing AAI operation independent of the vLP to collectively providing with the vLP coordinated dual chamber operation (e.g., DDD, VDD, DDI, VDI, or DOO operation) during which i2i messages are transmitted by the aLP and the vLP (to support the coordinated dual chamber operation), as indicated at step 620. While collectively providing the coordinated dual chamber operation, the aLP and the vLP transmit i2i messages to one another (e.g., on a beat-to-beat basis) or to a third IMD that acts as a communication hub for the aLP and the vLP.

At step 622, there is a determination of whether or not the aLP received a further mode switch type i2i message from the vLP. If the answer to the determination at step 622 is No, then flow returns to step 620 and the aLP continues to provide, collectively with the vLP, the coordinated dual chamber operation during which i2i messages are transmitted by the aLP and the vLP (to support the coordinated dual chamber operation). If the answer to the determination at step 622 is Yes, then flow goes to step 624.

At step 624 the aLP transmits a further mode switch acknowledgement (ACK) type i2i message to the vLP, and then flow returns to step 604, and the aLP returns to providing the AAI operation again. At this point the aLP can return to normally disabling its receiver(s), or a portion thereof, because beat-by-beat i2i communication need not be used during the AAI+VVI operation.

Referring now to the steps shown on the right in FIG. 6A, as noted above, at step 632 the vLP using its sense amplifier (e.g., 132) to sense a vEGM, which the vLP (and more specifically, the controller thereof) uses to detect intrinsic ventricular events, such as R-waves. As also noted above, step 634 involves the vLP providing the VVI operation. While the vLP provides the VVI operation at step 634, and the aLP simultaneously provides the AAI operation at step 604, independent of one another, the dual chamber LP system (e.g., 100) is collectively providing for the AAI+VVI operation in accordance with an embodiment of the present technology.

To reduce the probability of the vLP mistakenly detecting a paced atrial event as an intrinsic ventricular event, at step 636 the vLP monitors for possible crosstalk that may be caused by the aLP performing atrial pacing. Such possible crosstalk monitoring at step 636 can be performing, for example, using one of the techniques described in U.S. Patent No. 10,182,765, which was mentioned above. At step 638 there is a determination of whether or not the vLP detects possible crosstalk that may be caused by the aLP performing atrial pacing, and more specifically, the aLP delivering a pacing pulse. If the answer to the determination at step 638 is No, then step 640 is skipped and flow goes to step 642. If the answer to the determination at step 638 is Yes, then flow goes to step 640. At step 640 the vLP initiates providing crosstalk protection for a crosstalk protection period, so as to avoid crosstalk caused by an atrial pacing pulse from being mistakenly detected as an intrinsic ventricular event. The crosstalk protection period utilized at instances of step 640 can have the same duration as, or a different duration than, the crosstalk protection period utilized at instance of step 610, depending upon implementation. Such crosstalk protection can be performed in various different manners, examples of which were discussed above with reference to step 610, and thus need not be repeated. Instead of performing blanking at step 640, the vLP (and more specifically its controller) can ignore an intrinsic ventricular event that is detected during the crosstalk protection period, because it can be assumed that such a detected intrinsic ventricular event is actually crosstalk caused by the aLP performing atrial pacing. The blocks corresponding to steps 636, 638, and 640 in FIG. 6A are shown in dashed lines to show that such steps are optional, and that it is alternatively possible that flow goes directly from step 634 to step 642. It is also possible that just one of the vLP and the aLP monitors for possible crosstalk from the other. More specifically, it is possible that steps 636, 638, and 640 are performed, but steps 606, 608, and 610 are not performed. Alternatively, it is possible that steps 606, 608, and 610 are not performed, and steps 636, 638, and 640 are performed.

Still referring to FIG. 6A, at step 642 the vLP monitors for a first specified criterion, which if satisfied, would indicate that there should be a mode switch from the AAI+VVI operation to the coordinated dual chamber operation (e.g., one of DDD, VDD, DDI, VDI, or DOO operation). In accordance with certain embodiments, the first specified criterion, which the vLP determines whether or not is satisfied, comprises the vLP having provided at least a first specified threshold amount of ventricular pacing within a first specified duration. For example, the first specified criterion could be whether the vLP delivered ventricular pacing pulses during at least a specified percent (e.g., 70 percent, or some other percent) of most recent cardiac cycles, or during at least a specified number N (e.g., N = 7, or some other number) of cardiac cycles during the most recent number M (e.g., M = 10, or some other number > N) of cardiac cycles, or over some specified duration of time (e.g., 1 minute, 2 minutes, or 5 minutes, etc.). In certain embodiments, the vLP abstains from using its transmitter to transmit any i2i messages to the aLP (or to a third IMD that acts as a communication hub for the LPs) that are intended to be used by the aLP to coordinate pacing with the vLP while the vLP is providing the VVI operation. While the vLP abstains from using its transmitter to transmit such i2i messages to the aLP, the vLP may also disable or at least partially disable its receiver to further conserver power.

At step 644 there is a determination of whether or not the first specified criterion was satisfied. If the answer to the determination at step 644 is No, then flow returns to step 634 and the vLP continues to provide the VVI operation. This also results in the aLP continuing to provide the AAI operation. If the answer to the determination at step 644 is Yes, then flow goes to step 646. At step 646 that vLP transmits a mode switch type i2i message to the aLP. As part of step 646, or just prior to step 646, the vLP may enable a transmitter thereof (if the transmitter had been disabled to conserve power) to transmit i2i messages to the aLP, and the vLP may also enable one or more of its receiver(s) (if the receiver(s) thereof had been previously disabled to conserve power) to listen for a mode switch ACK i2i message that may be transmitted by the aLP. More generally, at step 644 there is a determination of whether the dual chamber LP system should switch from AAI+VVI operation to coordinated dual chamber operation when a patient's need for ventricular pace support becomes more than just rare or intermittent.

At step 648 that is a determination by the vLP (and more specifically, a controller thereof) of whether or not a mode switch ACK i2i message was received from the aLP by the vLP. If the answer to the determination at step 648 is No, then flow returns to step 646 and the vLP transmits another mode switch i2i message to the aLP. This can continue until the answer to the determination at step 648 is Yes, or until there is some timeout. If the answer to the determination at step 648 is Yes, then flow goes to step 650. At step 650 the vLP changes its mode of operation from providing VVI operation to collectively with the aLP providing coordinated dual chamber operation during which i2i messages are transmitted by the aLP and the vLP (to support the coordinated dual chamber operation). As noted above, while collectively providing the coordinated dual chamber operation, the aLP and the vLP transmit i2i messages (e.g., on a beat-to-beat basis) to enable coordinated dual chamber therapy. The coordinated dual chamber operation collectively provided by the aLP and the vLP at steps 620 and 650 may or may not be rate responsive, depending upon how the dual chamber LP system is programmed.

Still referring to FIG. 6A, at step 652 the vLP monitors for a second specified criterion, which if satisfied, would indicate that there should be a mode switch from the coordinated dual chamber operation back to the AAI+VVI operation. In accordance with certain embodiments, the second specified criterion, which the vLP determines whether or not is satisfied, comprises the vLP having provided less than a second specified threshold amount of ventricular pacing within a second specified duration. The second specified threshold used at step 652 can be the same as the first specified threshold used at step 642, or can be different than (e.g., less than) the first specified threshold used at step 642 to provide for hysteresis. For example, the second specified criterion could be whether the vLP delivered ventricular pacing pulses during less than a specified percent (e.g., 30 percent, or some other percent) of most recent cardiac cycles, or during less than a specified number N (e.g., N = 30, or some other number) of cardiac cycles during the most recent number M (e.g., M = 100, or some other number > N) of cardiac cycles, or over some specified duration of time (e.g., 1 minute, 2 minutes, or 5 minutes, etc.).

At step 654 there is a determination of whether or not the second specified criterion was satisfied. If the answer to the determination at step 654 is No, then flow returns to step 650 and the vLP continues to provide the coordinated dual chamber operation collectively with the aLP. If the answer to the determination at step 654 is Yes, then flow goes to step 656. At step 656 that vLP transmits a further mode switch type i2i message to the aLP (or to the third IMD that acts as a communication hub for the LPs). More generally, at step 654 there is a determination of whether the dual chamber LP system should switch from the coordinated dual chamber operation back to the AAI+VVI because the patient's need for ventricular pacing returned to be relatively rare or intermittent.

At step 658 there is a determination by the vLP (and more specifically, a controller thereof) of whether or not a further mode switch ACK i2i message was received from the aLP by the vLP. If the answer to the determination at step 658 is No, then flow returns to step 656 and the vLP transmits another further mode switch i2i message to the aLP. This can continue until the answer to the determination at step 658 is Yes, or until there is some timeout. If the answer to the determination at step 658 is Yes, then flow returns to step 634, and the vLP returns to providing the VVI operation. This also results in the aLP returning to providing the AAI operation, and thus, to the dual chamber LP system (e.g., 100) returning to providing the new AAI+VVI operation. In certain embodiments, in order to conserve power and thereby increase longevity of the aLP and the vLP, once the dual chamber LP system (e.g., 100) has returned to providing the AAI+VVI operation, the aLP abstains from transmitting any i2i messages that are intended to be used to coordinate operation of the vLP with the aLP while the vLP is providing the VVI operation, and the vLP abstains from transmitting any i2i messages that are intended to be used to coordinate operation of the aLP with the vLP while the aLP is providing the AAI operation. In other words, AAI+VVI operation, once returned to, is provided without requiring any i2i messages being sent from the aLP to the vLP (directly, or via a third IMD that acts as a communication hub), and vice versa.

In the embodiments described above with reference to FIG. 6A, the aLP and the vLP mode switch from providing AAI+VVI operation to collectively providing the coordinated dual chamber operation, and vice versa. As was explained above, the AAI operation may or may not be rate responsive, and the VVI operation may or may not be rate responsive, depending upon how the dual chamber LP system is programmed.

In accordance with certain embodiments of the present technology, while the aLP is providing the AAI operation and the vLP is providing the VVI operation, independent of one another, the aLP monitors for a specific criterion (referred to as a third specified criterion) that when detected by the aLP causes the aLP to send an i2i message to the vLP (or to a third IMD that acts as a communication hub for the LPs), which i2i message instructs the vLP to change (e.g., increase) its base pacing rate (e.g., from 40 ventricular beats per minute, to 60 ventricular beats per minute, but not limited thereto), or equivalently instructs the vLP to change (e.g., shorten) its base VV interval. For example, the third specified criterion can be that the aLP has detected that the atrial rate exceeds some specified threshold (e.g., 170 atrial beats per minute, but not limited thereto). In order for the vLP to be able to receive such an i2i message, the vLP should temporarily enable its receiver from time to time, in a similar manner to what the aLP does so at steps 612 and 614. The aLP can repeatedly send such a message until the aLP receives an ACK message that the vLP received the message, in a similar manner that the vLP does at steps 646 and 648. Thereafter, the aLP may monitor for a fourth criterion, e.g., that the atrial rate has fallen below the specified threshold or some further specified threshold (e.g., 130 atrial beats per minute, to provide hysteresis, but not limited thereto). In response to the aLP detecting the fourth criterion, the aLP sends an i2i message to the vLP (or to a third IMD that acts as a communication hub for the LPs), which i2i message instructs the vLP to change (e.g., reduce) its base pacing rate (e.g., from 60 ventricular beats per minute, back to 40 ventricular beats per minute, but not limited thereto), or equivalently instructs the vLP to change (e.g., increase) its base VV interval. The aLP can repeatedly send such an i2i message until the aLP receives an ACK message that the vLP received the message. This is similar to what the vLP does at steps 652, 654 656, and 658. A purpose of such an embodiment is to attempt to provide enhanced ventricular pacing support during atrial fibrillation (AF) and/or another type of atrial tachycardia, without requiring beat-to-beat i2i communication during the atrial tachycardia.

In certain embodiments, the aLP and the vLP collectively provide AAI+VVI operation without ever mode switching to collectively providing the coordinated dual chamber operation. The flow diagram in FIG. 6B shows how such a dual chamber LP system may operate. In such embodiments, the aLP and the vLP can completely abstain from transmitting i2i messages to one another, or may infrequently transmit i2i message to one another. As was the case with the embodiments summarized above, the AAI operation may or may not be rate responsive, and the VVI operation may or may not be rate responsive, depending upon how the dual chamber LP system is programmed. The blocks corresponding to steps 606, 608, and 610 in FIG. 6B are shown in dashed lines to show that such steps are optional. Similarly, the blocks corresponding to steps 636, 638, and 640 in FIG. 6B are shown in dashed lines to show that such steps are optional.

The steps in FIGS. 6A and 6B that are performed by the aLP can be performed under the control of the controller (e.g., 112) of the aLP by appropriately configuring the aLP using firmware and/or software thereof. Similarly, the steps in FIGS. 6A and 6B that are performed by the vLP can be performed under the control of the controller (e.g., 112) of the vLP by appropriately configuring the vLP using firmware and/or software thereof.

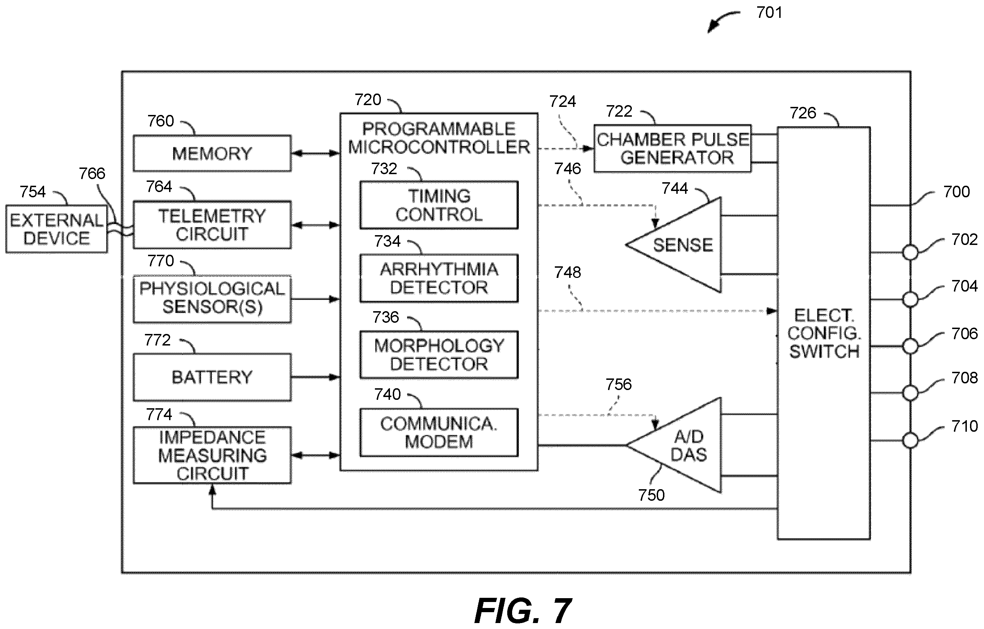

FIG. 7 shows a block diagram of one embodiment of an LP 701 (e.g., 102a or 102b) that is implanted into the patient as part of the implantable cardiac system in accordance with certain embodiments herein.

LP 701 has a housing 700 to hold the electronic/computing components. Housing 700 (which is often referred to as the "can", "case", "encasing", or "case electrode") may be programmably selected to act as the return electrode for certain stimulus modes. The LP is shown as including electrode terminals 702, 704, 706, 708, and 710, however the LP would likely include only two or three electrode terminals, depending upon how many electrodes the LP has.

The terminals may be connected to electrodes that are located in various locations on housing 700 or elsewhere within and about the heart. LP 701 includes a programmable microcontroller 720 that controls various operations of LP 701, including cardiac monitoring and stimulation therapy. Microcontroller 720 includes a microprocessor (or equivalent control circuitry), RAM and/or ROM memory, logic and timing circuitry, state machine circuitry, and I/O circuitry.

LP 701 further includes a pulse generator 722 that generates stimulation pulses and communication pulses for delivery by one or more electrodes coupled thereto. Pulse generator 722 is controlled by microcontroller 720 via control signal 724. Pulse generator 722 may be coupled to the select electrode(s) via an electrode configuration switch 726, which includes multiple switches for connecting the desired electrodes to the appropriate I/O circuits, thereby facilitating electrode programmability. Switch 726 is controlled by a control signal 728 from microcontroller 720.

In the embodiment of FIG. 7, a single pulse generator 722 is illustrated. Optionally, the IMD may include multiple pulse generators, similar to pulse generator 722, where each pulse generator is coupled to one or more electrodes and controlled by microcontroller 720 to deliver select stimulus pulse(s) to the corresponding one or more electrodes.

Microcontroller 720 is illustrated as including timing control circuitry 732 to control the timing of the stimulation pulses (e.g., pacing rate, atrio-ventricular (AV) delay, atrial interconduction (AA) delay, or ventricular interconduction (VV) delay, etc.). The AA delay can also be referred to as an AA interval, and the VV delay can also be referred to as the VV interval. Timing control circuitry 732 may also be used for the timing of refractory periods, blanking intervals, noise detection windows, evoked response windows, alert intervals, marker channel timing, and so on. Microcontroller 720 also has an arrhythmia detector 734 for detecting arrhythmia conditions. Microcontroller 720 also has an AV synchrony detector 736 that can be configured to determine an AV synchrony metric in accordance with embodiments of the present technology described herein. Although not shown, the microcontroller 720 may further include other dedicated circuitry and/or firmware/software components that assist in monitoring various conditions of the patient's heart and managing pacing therapies. The microcontroller can include a processor. The microcontroller, and/or the processor thereof, can be used to perform the methods described herein.

LP 701 is further equipped with a communication modem (modulator/demodulator) 740 to enable wireless communication with the remote slave pacing unit. Modem 740 may include one or more transmitters and two or more receivers as discussed herein in connection with FIG. 1B. In one implementation, modem 740 may use low or high frequency modulation. As one example, modem 740 may transmit i2i messages and other signals through conducted communication between a pair of electrodes. Modem 740 may be implemented in hardware as part of microcontroller 720, or as software/firmware instructions programmed into and executed by microcontroller 720. Alternatively, modem 740 may reside separately from the microcontroller as a standalone component.

LP 701 includes a sensing circuit 744 selectively coupled to one or more electrodes, that perform sensing operations, through switch 726 to detect the presence of cardiac activity in the right chambers of the heart. Sensing circuit 744 may include dedicated sense amplifiers, multiplexed amplifiers, or shared amplifiers. It may further employ one or more low power, precision amplifiers with programmable gain and/or automatic gain control, bandpass filtering, and threshold detection circuit to selectively sense the cardiac signal of interest. The automatic gain control enables the unit to sense low amplitude signal characteristics of atrial fibrillation. Switch 726 determines the sensing polarity of the cardiac signal by selectively closing the appropriate switches. In this way, the clinician may program the sensing polarity independent of the stimulation polarity.

The output of sensing circuit 744 is connected to microcontroller 720 which, in turn, triggers or inhibits the pulse generator 722 in response to the presence or absence of cardiac activity. Sensing circuit 744 receives a control signal 746 from microcontroller 720 for purposes of controlling the gain, threshold, polarization charge removal circuitry (not shown), and the timing of any blocking circuitry (not shown) coupled to the inputs of the sensing circuitry.

In the embodiment of FIG. 7, a single sensing circuit 744 is illustrated. Optionally, the IMD may include multiple sensing circuits, similar to sensing circuit 744, where each sensing circuit is coupled to one or more electrodes and controlled by microcontroller 720 to sense electrical activity detected at the corresponding one or more electrodes. Sensing circuit 744 may operate in a unipolar sensing configuration or in a bipolar sensing configuration.

LP 701 further includes an analog-to-digital (A/D) data acquisition system (DAS) 750 coupled to one or more electrodes via switch 726 to sample cardiac signals across any pair of desired electrodes. Data acquisition system 750 is configured to acquire intracardiac electrogram signals, convert the raw analog data into digital data, and store the digital data for later processing and/or telemetric transmission to an external system 754 (e.g., a programmer, local transceiver, or a diagnostic system analyzer). Data acquisition system 750 is controlled by a control signal 756 from the microcontroller 720.

Microcontroller 720 is coupled to a memory 760 by a suitable data/address bus. The programmable operating parameters used by microcontroller 720 are stored in memory 760 and used to customize the operation of LP 701 to suit the needs of a particular patient. Such operating parameters define, for example, pacing pulse amplitude, pulse duration, electrode polarity, rate, sensitivity, automatic features, arrhythmia detection criteria, and the amplitude, waveshape and vector of each stimulation pulse to be delivered to the patient's heart. The memory 760 can also store instructions for performing at least some of the steps of the methods described above with reference to FIGS. 6A and 6B.

The operating parameters of LP 701 may be non-invasively programmed into memory 760 through a telemetry circuit 764 in telemetric communication via communication link 766 with external system 754. Telemetry circuit 764 allows intracardiac electrograms and status information relating to the operation of LP 701 (as contained in microcontroller 720 or memory 760) to be sent to external system 754 through communication link 766.

LP 701 can further include magnet detection circuitry (not shown), coupled to microcontroller 720, to detect when a magnet is placed over the unit. A magnet may be used by a clinician to perform various test functions of LP 701 and/or to signal microcontroller 720 that external system 754 is in place to receive or transmit data to microcontroller 720 through telemetry circuits 764.

LP 701 can further include one or more physiological sensors 770. Such sensors are commonly referred to as "rate-responsive" sensors because they are typically used to adjust pacing stimulation rates according to the exercise state of the patient. However, physiological sensor 770 may further be used to detect changes in cardiac output, changes in the physiological condition of the heart, or diurnal changes in activity (e.g., detecting sleep and wake states). Signals generated by physiological sensors 770 are passed to microcontroller 720 for analysis. Microcontroller 720 responds by adjusting the various pacing parameters (such as rate, AV Delay, VV Delay, etc.) at which the atrial and ventricular pacing pulses are administered. While shown as being included within LP 701, physiological sensor(s) 770 may be external to LP 701, yet still be implanted within or carried by the patient. Examples of physiologic sensors might include sensors that, for example, sense respiration rate, pH of blood, ventricular gradient, activity, position/posture, minute ventilation (MV), and so forth.

A battery 772 provides operating power to all of the components in LP 701. Battery 772 is capable of operating at low current drains for long periods of time. Battery 772 also desirably has a predictable discharge characteristic so that elective replacement time can be detected. As one example, LP 701 employs lithium/silver vanadium oxide batteries.

LP 701 further includes an impedance measuring circuit 774, which can be used for many things, including: lead impedance surveillance during the acute and chronic phases for proper lead positioning or dislodgement; detecting operable electrodes and automatically switching to an operable pair if dislodgement occurs; measuring respiration or minute ventilation; detecting when the device has been implanted; measuring stroke volume; and detecting the opening of heart valves; and so forth. Impedance measuring circuit 774 is coupled to switch 726 so that any desired electrode may be used.

An aspect of the embodiments relate to a dual chamber LP system, comprising an atrial leadless pacemaker (aLP) and a ventricular leadless pacemaker (vLP). The aLP is configured to be implanted in or on an atrial cardiac chamber of a patient's heart. The vLP is configured to be implanted in or on a ventricular cardiac chamber of the patient's heart. The aLP is configured to provide AAI operation, and the vLP is configured to provide VVI operation, during a same period of time and independent of one another. While the aLP provides the AAI operation, the aLP performs atrial pacing when an intrinsic atrial event is not detected within a specified AA interval following a previous paced or intrinsic atrial event, performs atrial sensing, and inhibits the atrial pacing when the intrinsic atrial event is detected within the specified AA interval following the previous paced or intrinsic atrial event. While the vLP provides the VVI operation, the vLP performs ventricular pacing when an intrinsic ventricular event is not detected within a specified VV interval following a previous paced or intrinsic ventricular event, performs ventricular sensing, and inhibits the ventricular pacing when the intrinsic ventricular event is detected within the specified VV interval following the previous paced or intrinsic ventricular event.

In an embodiment, each LP, of the aLP and the vLP, includes a respective transmitter that enables the LP to transmit i2i messages, includes a respective receiver that enables the LP to receive i2i messages, and includes a respective power source (e.g., battery) used to power the transmitter and the receiver of the LP. In certain such embodiments, during the same first period of time that the aLP provides the AAI operation and the vLP provides the VVI operation independent of one another, the aLP is configured to abstain from transmitting any i2i messages that are intended to be used to coordinate operation of the vLP with the aLP, and the vLP is configured to abstain from transmitting any i2i messages to that are intended to be used to coordinate operation of the aLP with the vLP.

In an embodiment, the specified AA interval is shorter than the specified VV interval to cause an atrial rate of the atrial pacing performed by the aLP to be faster than a ventricular rate of the ventricular pacing performed by the vLP.

In an embodiment, the aLP includes a sensor that enables the aLP to monitor an activity level of a patient within which the aLP and the vLP are implanted. Further, each LP, of the aLP and the vLP, includes a respective controller. In certain such embodiments, the controller of the aLP is configured to adjust an atrial rate of the atrial pacing performed by the aLP by adjusting the specified AA interval based on the activity level of the patient as detected using the sensor of the aLP while the aLP is providing the AAI operation. Additionally, the controller of the vLP is configured to not adjust a ventricular rate of the ventricular pacing performed by the vLP while the vLP is providing the VVI operation. In other embodiments, the controller of the aLP is configured to adjust an atrial rate of the atrial pacing performed by the aLP by adjusting the specified AA interval based on the activity level of the patient as detected using the sensor of the aLP while the aLP is providing the AAI operation, and the controller of the vLP is configured to adjust a ventricular rate of the ventricular pacing performed by the vLP by adjusting the VV interval based on the activity level of the patient as detected using the sensor of the vLP while the vLP is providing the VVI pacing, wherein such an embodiment can be referred to more specifically as AAIR+VVIR operation. In such embodiments, the vLP includes a sensor that enables the vLP to monitor the activity level of the patient.

In an embodiment, the aLP includes a sense amplifier configured to sense an atrial electrogram (aEGM) used by the aLP to detect intrinsic atrial events, and the vLP includes a sense amplifier configured to sense a ventricular electrogram (vEGM) used by the vLP to detect intrinsic ventricular events. Additionally, the aLP includes a controller that is communicatively coupled to the sense amplifier of the aLP, and the vLP includes a controller that is communicatively coupled to the sense amplifier of the vLP. In certain such embodiments, the controller of the vLP is configured to monitor for possible crosstalk that may be caused by the aLP performing the atrial pacing, while the vLP is providing the VVI operation, and initiate providing crosstalk protection for a first crosstalk protection period, in response to the controller of the vLP detecting possible crosstalk that may be caused by the aLP performing the atrial pacing. Additionally, or alternatively, the controller of the aLP is configured to monitor for possible crosstalk that may be caused by the vLP performing the ventricular pacing, while the aLP is providing the AAI operation, and initiate providing crosstalk protection for a second crosstalk protection period, in response to the controller of the aLP detecting possible crosstalk that may be caused by the vLP performing the ventricular pacing.

In an embodiment, each LP, of the aLP and the vLP, includes a respective transmitter that enables the LP to transmit i2i messages to the other LP, includes a respective receiver that enables the LP to receive i2i messages from the other LP, and includes a respective battery used to power the transmitter and the receiver of the LP. Additionally, the aLP includes a controller that is communicatively coupled to the transmitter and the receiver thereof and is powered by the battery thereof, and the vLP includes a controller that is communicatively coupled to the transmitter and the receiver thereof and is powered by the battery thereof. In certain such embodiments, the controller of the vLP is configured to determine whether or not a first specified criterion is satisfied, abstain from using the transmitter of the vLP to transmit any i2i messages that are intended to be used to coordinate operation of the aLP with the vLP during a first period of time when the first specified criterion is not satisfied, and cause the transmitter of the vLP to transmit a mode switch type of i2i message in response to the first specified criterion being satisfied. The mode switch type of i2i message, if successfully received by and acknowledged by the aLP, causes the aLP and the vLP to transition (from respectively providing the AAI operation and the VVI operation, independent of one another) to collectively providing coordinated dual chamber operation during a second period of time, during which i2i messages are transmitted by the aLP and the vLP (to support the coordinated dual chamber operation). In certain embodiments, collectively providing coordinated dual chamber operation, during which i2i messages are transmitted by the aLP and the vLP, comprises collectively providing DDD operation. In other words, in specific embodiments the coordinated dual chamber operation is DDD operation. The aLP and the vLP collectively providing coordinated dual chamber operation, during which i2i messages are transmitted by the aLP and the vLP (to support the coordinated dual chamber operation), can alternatively comprise collectively providing VDD, DDI, VDI, or DOO operation.

In an embodiment, the controller of the aLP is configured to normally disable at least a portion of the receiver of the aLP that enables the aLP to receive one or more i2i messages to thereby conserve power of the power source (e.g., battery) of the aLP compared to if the at least the portion of the receiver of the aLP were enabled. Additionally, the controller of the aLP is configured to from time to time temporarily enable the at least the portion of the receiver of the aLP, that enables the aLP to receive one or more i2i messages, to thereby enable the aLP to monitor for the mode switch type of i2i message that causes the aLP to mode switch from providing the AAI operation to collectively providing with the vLP the coordinated dual chamber operation, during which i2i messages are transmitted by the aLP and the vLP (to support the coordinated dual chamber operation). The controller of the aLP is also configured to cause the aLP to mode switch from providing the AAI operation to collectively providing with the vLP the coordinated dual chamber operation, in response to the aLP receiving and acknowledging the mode switch type of i2i message. In certain such embodiments, the controller of the aLP causes the aLP to abstain from using its transmitter to transmit any i2i messages that are intended to be used to coordinate operation of the vLP with the aLP until the aLP receives a mode switch type of i2i message from the vLP.

In an embodiment, the first specified criterion, which the controller of the vLP is configured to determine whether or not is satisfied, comprises the vLP having provided at least a first specified threshold amount of ventricular pacing within a first specified duration.

In an embodiment, the controller of the vLP is configured to determine whether or not a second specified criterion is satisfied, after the vLP has mode switched from providing the VVI operation to collectively providing with the aLP the coordinated dual chamber operation. The controller of the vLP is also configured to, during a third period of time that begins in response to the second specified criterion being satisfied, use the transmitter of the vLP to transmit a further mode switch type of i2i message to the aLP, which if successfully received by and acknowledged by the aLP causes the aLP to mode switch from collectively providing with the vLP the coordinated dual chamber operation to the aLP again providing the AAI operation independent of the vLP, and causes the vLP to mode switch from collectively providing with the aLP the coordinated dual chamber operation to the vLP again providing the VVI operation independent of the aLP. In other words, the second specified criterion when satisfied causes a transition from the coordinated dual chamber operation back to the AAI+VVI operation.

In an embodiment, the second specified criterion, which the controller of the vLP is configured to determine whether or not is satisfied, comprises the vLP having provided less than a second specified threshold amount of ventricular pacing within a second specified duration.

In an embodiment, while the aLP is providing the AAI operation and the vLP is providing the VVI operation, independent of one another, the controller of the aLP is configured to monitor for a third specific criterion that when detected causes the transmitter of the aLP to send an i2i message to the vLP, or to a third IMD that acts as a communication hub for the LPs, instructing the vLP to change its base pacing rate or its base VV interval.

In an embodiment, the third specified criterion, which the controller of the aLP is configured to determine whether or not is satisfied, comprises the aLP having detected that the atrial rate exceeds a specified threshold.

Another aspects of the embodiments relates to a method for use with a dual chamber leadless pacemaker (LP) system including an atrial leadless pacemaker (aLP) and a ventricular leadless pacemaker (vLP). The method comprises, during a same first period of time, during which the aLP and the vLP abstain from communicating with one another, the aLP providing AAI operation and the vLP providing VVI operation independent of one another. The method also comprises, during a second period of time, that begins in response to a first specified criterion being satisfied, the vLP and the aLP communicating with one another and mode switching, from the aLP providing AAI operation and the vLP providing VVI operation independent of one another, to the aLP and the vLP collectively providing one of DDD, VDD, DDI, VDI, or DOO operation.

In an embodiment, the method further comprises, during a third period of time that begins in response to a second specified criterion being satisfied, the aLP and the vLP mode switching back from collectively providing the one of DDD, VDD, DDI, VDI, or DOO operation, to the vLP again providing the VVI operation and vLP again providing the VVI operation independent of one another.

In an embodiment, the first specified criterion is satisfied when the vLP provides at least a first specified threshold amount of ventricular pacing within a first specified duration, and the second specified criterion is satisfied when the vLP provided less than a second specified threshold amount of ventricular pacing within a second specified duration.

Another aspects of the embodiments relates to a method for use with a dual chamber leadless pacemaker (LP) system including an atrial leadless pacemaker (aLP) and a ventricular leadless pacemaker (vLP). The method comprises the aLP providing AAI operation, during which the aLP performs atrial pacing when an intrinsic atrial event is not detected within a specified AA interval following a previous paced or intrinsic atrial event, performs atrial sensing, and inhibits the atrial pacing when the intrinsic atrial event is detected within the specified AA interval following the previous paced or intrinsic atrial event. The method also comprises the vLP providing VVI operation, during which the vLP performs ventricular pacing when an intrinsic ventricular event is not detected within a specified VV interval following a previous paced or intrinsic ventricular event, performs ventricular sensing, and inhibits the ventricular pacing when the intrinsic ventricular event is detected within the specified VV interval following the previous paced or intrinsic ventricular event. In this method, the aLP providing the AAI operation, and the vLP providing the VVI operation, occurs during a same first period of time and independent of one another. During this first period of time the aLP and the vLP of the dual chamber LP system collectively provide what is referred to herein as AAI+VVI operation.

In an embodiment, each LP, of the aLP and the vLP, includes a respective transmitter that enables the LP to transmit i2i messages (to the other LP, or to a third IMD that acts as a communication hub for the LPs), includes a respective receiver that enables the LP to receive i2i messages (from the other LP, or from the third IMD that acts as a communication hub for the LPs), and includes a respective power source (e.g., battery) used to power the transmitter and the receiver of the LP. In certain such embodiments, the method further comprises, during the same first period of time that the aLP provides the AAI operation and the vLP provides the VVI operation independent of one another, the aLP abstaining from transmitting any i2i messages that are intended to be used to coordinate operation of the vLP with the aLP, and the vLP abstaining from transmitting any i2i messages that are intended to be used to coordinate operation of the aLP with the vLP. This conserves power and thereby increases the longevity of the LPs.

In an embodiment, the specified AA interval is shorter than the specified VV interval to cause an atrial rate of the atrial pacing performed by the aLP to be faster than a ventricular rate of the ventricular pacing performed by the vLP.

In an embodiment, the aLP includes a sensor that enables the aLP to monitor an activity level of a patient within which the aLP and the vLP are implanted. In certain such embodiments, while the aLP is providing the AAI operation and the vLP is providing the VVI operation, the aLP adjusts an atrial rate of the atrial pacing performed by the aLP by adjusting the specified AA interval based on the activity level of the patient as detected using the sensor of the aLP, and the vLP does not adjust a ventricular rate of the ventricular pacing performed by the vLP, wherein such an embodiment can be referred to more specifically as AAIR+VVI operation. In other embodiments, while the aLP is providing the AAI operation and the vLP is providing the VVI operation, the aLP adjusts an atrial rate of the atrial pacing performed by the aLP by adjusting the specified AA interval based on the activity level of the patient as detected using the sensor of the aLP, and the vLP adjusts a ventricular rate of the ventricular pacing performed by the vLP by adjusting the VV interval based on the activity level of the patient as detecting using the sensor of the vLP, wherein such an embodiment can be referred to more specifically as AAIR+VVIR operation. In such embodiments, the vLP also includes a sensor that enables the vLP to monitor the activity level of the patient.

In an embodiment, each LP, of the aLP and the vLP, includes a respective sense amplifier, wherein the sense amplifier of the aLP is configured to sense an atrial electrogram (aEGM) used to detect intrinsic atrial events, and the sense amplifier of the vLP is configured to sense a ventricular electrogram (vEGM) used to detect intrinsic ventricular events. In certain such embodiments, while the aLP is providing the AAI operation and the vLP is providing the VVI operation, the method further comprises the aLP monitoring for possible crosstalk that may be caused by the vLP performing the ventricular pacing and initiating providing crosstalk protection for a first crosstalk protection period, in response to the aLP detecting possible crosstalk that may be caused by the vLP performing the ventricular pacing. Additionally, or alternatively, the method can include the vLP monitoring for possible crosstalk that may be caused by the aLP performing the atrial pacing and the vLP initiating providing crosstalk protection for a second crosstalk protection period, in response to the vLP detecting possible crosstalk that may be caused by the aLP performing the atrial pacing. The second crosstalk protection period may have a same duration as, or a different duration than, the first crosstalk protection period, depending upon the implementation.

In an embodiment, the method further comprises the vLP determining whether or not a first specified criterion is satisfied, and the vLP, during a first period of time when the first specified criterion is not satisfied, abstaining from using the transmitter of the vLP to transmit any i2i messages that are intended to be used to coordinate operation of the aLP with the vLP. While the vLP abstains from using its transmitter to transmit any i2i messages that are intended to be used to coordinate operation of the aLP with the vLP , the vLP may also disable or at least partially disable its receiver to further conserver power. The method also comprises the vLP, during a second period of time that begins in response to the first specified criterion being satisfied, using the transmitter of the vLP to transmit a mode switch type of i2i message to the aLP, which if successfully received by and acknowledged by the aLP, causes aLP and the vLP to transition (from respectively providing the AAI operation and the VVI operation) to collectively providing coordinated dual chamber operation (which can be any one of DDD, VDD, DDI, VDI, and DOO operation), during which i2i messages are transmitted by the aLP and the vLP (to support the coordinated dual chamber operation). In specific embodiments, the coordinated dual chamber operation is DDD operation, during which the aLP and the vLP transmit i2i messages (e.g., on a beat-to-beat basis) to enable coordinated dual chamber pacing, and during which the vLP performs ventricular pacing when an intrinsic ventricular event is not detected within a specified atrioventricular (AV) delay following a previous paced or intrinsic atrial event, performs ventricular sensing, and inhibits the ventricular pacing when the intrinsic ventricular event is detected within the specified AV delay following the previous paced or intrinsic atrial event. In accordance with other embodiments, the coordinated dual chamber operation comprises one of VDD, DDI, VDI, or DOO operation.

In an embodiment, the method includes the aLP normally disabling at least a portion of the respective receiver of the aLP that enables the aLP to receive one or more i2i messages to thereby conserve power of the power source (e.g., battery) of the aLP compared to if the at least the portion of the respective receiver of the aLP was enabled. In certain such embodiments, the method further comprises the aLP from time to time temporarily enabling at least the portion of the receiver of the aLP that enables the aLP to receive one or more i2i messages from the vLP (or from a third IMD that acts as a communication hub for the LPs), to thereby enable the aLP to monitor for the mode switch type of i2i message. The method also includes the aLP receiving the mode switch type of i2i message from the vLP (or a third IMD that acts as a communication hub for the LPs) and in response thereto transmitting a mode switch acknowledgement message and collectively providing with the vLP the coordinated dual chamber operation during which i2i messages transmitted by the aLP and the vLP (to support the coordinated dual chamber operation).

In certain such embodiments, the method further comprises the aLP abstaining from using its transmitter to transmit any i2i messages that are intended to be used to coordinate operation of the vLP with the aLP until the aLP receives a mode switch type of i2i message.

In an embodiment, the first specified criterion, which the vLP determines whether or not is satisfied, comprises the vLP having provided at least a first specified threshold amount of ventricular pacing within a first specified duration. The first specified threshold amount can be a first specified number, or a first specified percentage, but is not limited thereto. It is also possible that the first specified criterion, which the vLP determines whether or not is satisfied, comprises the vLP having delivered ventricular pacing during at least X of the Y most recent cardiac cycles, wherein X and Y are predetermined values and X < Y.

In an embodiment, the method further comprises, after the vLP has mode switched from providing the VVI operation to collectively providing with the aLP the coordinated dual chamber operation during which i2i messages transmitted by the aLP and the vLP (to support the coordinated dual chamber operation), the vLP determining whether or not a second specified criterion is satisfied. In certain such embodiments, the vLP, during a third period of time that begins in response to the second specified criterion being satisfied, using the transmitter of the vLP to transmit a further mode switch type of i2i message, which if successfully received by and acknowledged by the aLP causes the aLP to mode switch from collectively providing with the vLP the coordinated dual chamber operation to the aLP again providing the AAI operation independent of the vLP, and causes the vLP to mode switch from collectively providing with the aLP the coordinated dual chamber operation to the vLP again providing the VVI operation independent of the aLP. In other words, the second specified criterion when satisfied causes a transition from the coordinated dual chamber operation back to the AAI+VVI operation.

In an embodiment, the second specified criterion, which the vLP determines whether or not is satisfied, comprises the vLP having provided less than a second specified threshold amount of ventricular pacing within a second specified duration. The second specified threshold amount can be a second specified number, or a second specified percentage, but is not limited thereto. It is also possible that the second specified criterion, which the vLP determines whether or not is satisfied, comprises the vLP having delivered ventricular pacing during less than A of the B most recent cardiac cycles, wherein A and B are predetermined values and A < B.

In an embodiment of the present technology, while the aLP is providing the AAI operation and the vLP is providing the VVI operation, independent of one another, the aLP monitors for a third specified criterion that when detected by the aLP causes the aLP to send an i2i message to the vLP (or to a third IMD that acts as a communication hub for the LPs), which i2i message instructs the vLP to change (e.g., increase) its base pacing rate, or equivalently instructs the vLP to change (e.g., shorten) its base VV interval. In order for the vLP to be able to receive such an i2i message, the vLP should temporarily enable its receiver from time to time. The aLP can repeatedly send such a message until the aLP receives an ACK message that the vLP received the message. Thereafter, the aLP may monitor for a fourth criterion, e.g., that the atrial rate has fallen below the specified threshold or some further specified threshold. In response to the aLP detecting the fourth criterion, the aLP sends an i2i message to the vLP (or to a third IMD that acts as a communication hub for the LPs), which i2i message instructs the vLP to change (e.g., reduce) its base pacing rate, or equivalently instructs the vLP to change (e.g., increase) its base VV interval. The aLP can repeatedly send such an i2i message until the aLP receives an ACK message that the vLP received the message.

## Claims

1. A dual chamber leadless pacemaker, LP, system (100), comprising:
an atrial leadless pacemaker, aLP, (102a) configured to be implanted in or on an atrial cardiac chamber of a patient's heart (101); and
a ventricular leadless pacemaker, vLP, (102b) configured to be implanted in or on a ventricular cardiac chamber of the patient's heart (101);
each leadless pacemaker, LP, of the aLP (102a) and the vLP (102b), includes a respective transmitter (116, 722) that enables the LP to transmit implant-to-implant (i2i) messages, includes a respective receiver (120, 122) that enables the LP to receive i2i messages, and includes a respective power source (114, 772) used to power the transmitter (116, 722) and the receiver (120, 122) of the LP;
wherein the aLP (102a) is configured to provide AAI operation, and the vLP (102b) is configured to provide VVI operation;
wherein while the aLP (102a) provides the AAI operation, the aLP (102a) performs atrial pacing when an intrinsic atrial event is not detected within a specified AA interval following a previous paced or intrinsic atrial event, performs atrial sensing, and inhibits the atrial pacing when the intrinsic atrial event is detected within the specified AA interval following the previous paced or intrinsic atrial event; and
wherein while the vLP (102b) provides the VVI operation, the vLP (102b) performs ventricular pacing when an intrinsic ventricular event is not detected within a specified VV interval following a previous paced or intrinsic ventricular event, performs ventricular sensing, and inhibits the ventricular pacing when the intrinsic ventricular event is detected within the specified VV interval following the previous paced or intrinsic ventricular event, **characterized in that**
the aLP (102a) is configured to provide the AAI operation, and the vLP (102b) is configured to provide the VVI operation, during a same period of time and independent of one another; and
during the same period of time that the aLP (102a) provides the AAI operation and the vLP (102b) provides the VVI operation independent of one another, the aLP (102a) is configured to abstain from transmitting any i2i messages intended to be used to coordinate operation of the vLP (102b) with the aLP (102a), and the vLP (102b) is configured to abstain from transmitting any i2i messages intended to be used to coordinate operation of the aLP (102a) with the vLP (102b).

2. The system (100) of any one of claims 1, wherein:
the specified AA interval is shorter than the specified VV interval to cause an atrial rate of the atrial pacing performed by the aLP (102a) to be faster than a ventricular rate of the ventricular pacing performed by the vLP (102b).

3. The system (100) of any one of claims 1 through 2, wherein:
the aLP (102a) includes a sensor (152, 154) that enables the aLP (102a) to monitor an activity level of a patient within which the aLP (102a) and the vLP (102b) are implanted;
each LP, of the aLP (102a) and the vLP (102b), includes a respective controller (112, 720);
the controller (112, 720) of the aLP (102a) is configured to adjust an atrial rate of the atrial pacing performed by the aLP (102a) by adjusting the specified AA interval based on the activity level of the patient as detected using the sensor (152, 154) of the aLP (102a) while the aLP (102a) is providing the AAI operation; and
the controller (112, 720) of the vLP (102b) is configured to not adjust a ventricular rate of the ventricular pacing performed by the vLP (102b) while the vLP (102b) is providing the VVI operation.

4. The system (100) of any one of claims 1 through 3, wherein:
the aLP (102a) includes a sense amplifier (132, 744) configured to sense an atrial electrogram, aEGM, used by the aLP (102a) to detect intrinsic atrial events;
the vLP (102b) includes a sense amplifier (132, 744) configured to sense a ventricular electrogram, vEGM, used by the vLP (102b) to detect intrinsic ventricular events;
the aLP (102a) includes a controller (112, 720) that is communicatively coupled to the sense amplifier (132, 744) of the aLP (102a);
the vLP (102b) includes a controller (112, 720) that is communicatively coupled to the sense amplifier (132, 744) of the vLP (102b); and
the controller (112, 720) of the vLP (102b) is configured to monitor for possible crosstalk that may be caused by the aLP (102a) performing the atrial pacing, while the vLP (102b) is providing the VVI operation, and initiate providing crosstalk protection for a first crosstalk protection period, in response to detecting possible crosstalk that may be caused by the aLP (102a) performing the atrial pacing.

5. The system (100) of claim 4, wherein:
the controller (112, 720) of the aLP (102a) is configured to monitor for possible crosstalk that may be caused by the vLP (102b) performing the ventricular pacing, while the aLP (102a) is providing the AAI operation, and initiate providing crosstalk protection for a second crosstalk protection period, in response to detecting possible crosstalk that may be caused by the vLP (102b) performing the ventricular pacing.

6. The system (100) of any one of claims 1 through 5, wherein:
each leadless pacemaker, LP, of the aLP (102a) and the vLP (102b), includes a respective transmitter (116, 722) that enables the LP to transmit implant-to-implant (i2i) messages, includes a respective receiver (120, 122) that enables the LP to receive i2i messages, and includes a respective battery (114, 772) used to power the transmitter (116, 722) and the receiver (120, 122) of the LP;
the aLP (102a) includes a controller (112, 720) that is communicatively coupled to the transmitter (116, 722) and the receiver (120, 122) thereof and is powered by the battery (114, 772) thereof;
the vLP (102b) includes a controller (112, 720) that is communicatively coupled to the transmitter (116, 722) and the receiver (120, 122) thereof and is powered by the battery (114, 772) thereof;
the controller (112, 720) of the vLP (102b) is configured to determine whether or not a first specified criterion is satisfied, abstain from using the transmitter (116, 722) of the vLP (102b) to transmit any i2i messages that are intended to be used by the aLP (102a) to coordinate operation with the vLP (102b) during a first period of time when the first specified criterion is not satisfied, and cause the transmitter (116, 722) of the vLP (102b) to transmit a mode switch type of i2i message in response to the first specified criterion being satisfied; and
the mode switch type of i2i message, if successfully received by and acknowledged by the aLP (102a), causes the aLP (102a) and the vLP (102b) to collectively provide coordinated dual chamber operation.

7. The system (100) of claim 6, wherein the controller (112, 720) of the aLP (102a) is configured to:
normally disable at least a portion of the receiver (120, 122) of the aLP (102a) that enables the aLP (102a) to receive one or more i2i messages from the vLP (102b) to thereby conserve power of the battery (114, 772) of the aLP (102a) compared to if the at least the portion of the receiver (120, 122) of the aLP (102a) were enabled;
from time to time temporarily enable the at least the portion of the receiver (120, 122) of the aLP (102a), that enables the aLP (102a) to receive one or more i2i messages from the vLP (102b), to thereby enable the aLP (102a) to monitor for the mode switch type of i2i message that causes the aLP (102a) to mode switch from providing the AAI operation to collectively providing with the vLP (102b) the coordinated dual chamber operation; and
cause the aLP (102a) to mode switch from providing the AAI operation to collectively providing with the vLP (102b) the coordinated dual chamber operation, in response to the aLP (102a) receiving and acknowledging the mode switch type of i2i message from the vLP (102b).

8. The system (100) of any one of claims 6 through 7, wherein:
the first specified criterion, which the controller (112, 720) of the vLP (102b) is configured to determine whether or not is satisfied, comprises the vLP (102b) having provided at least a first specified threshold amount of ventricular pacing within a first specified duration.

9. The system (100) of claim 8, wherein the controller (112, 720) of the vLP (102b) is configured to:
determine whether or not a second specified criterion is satisfied, after the vLP (102b) has mode switched from providing the VVI operation to collectively providing with the aLP (102a) the coordinated dual chamber operation; and
during a third period of time that begins in response to the second specified criterion being satisfied, use the transmitter (116, 722) of the vLP (102b) to transmit a further mode switch type of i2i message to the aLP (102a), which if successfully received by and acknowledged by the aLP (102a) causes the aLP (102a) to mode switch from collectively providing with the vLP (102b) the coordinated dual chamber operation to the aLP (102a) again providing the AAI operation independent of the vLP (102b), and causes the vLP (102b) to mode switch from collectively providing with the aLP (102a) the coordinated dual chamber operation to the vLP (102b) again providing the VVI operation independent of the aLP (102a).

10. The system (100) of claim 9, wherein:
the second specified criterion, which the controller (112, 720) of the vLP (102b) is configured to determine whether or not is satisfied, comprises the vLP (102b) having provided less than a second specified threshold amount of ventricular pacing within a second specified duration.

11. The system (100) of claim 10, wherein:
while the aLP is providing the AAI operation and the vLP is providing the VVI operation, independent of one another, the controller (112, 720) of the aLP (102a) is configured to monitor for a third specific criterion that when detected causes the transmitter (116, 722) of the aLP (102a) to send an i2i message to the vLP (102b), or to a third IMD that acts as a communication hub for the LPs, instructing the vLP (102b) to change its base pacing rate or its base VV interval.

12. The system (100) of claim 11, wherein:
the third specified criterion, which the controller (112, 720) of the aLP (102a) is configured to determine whether or not is satisfied, comprises the aLP (102a) having detected that the atrial rate exceeds a specified threshold.

13. The system (100) of any one of claims 6 through 11, wherein the coordinated dual chamber operation comprises DDD operation.

14. The system (100) of any one of claims 6 through 12, wherein the coordinated dual chamber operation comprises one of VDD, DDI, VDI, or DOO operation.

## Patentansprüche

1. Zweikammer-Herzschrittmacher-System (100) ohne Elektroden, Zweikammer-LP-System, Folgendes umfassend:
einen atrialer Herzschrittmacher ohne Elektroden, aLP, (102a), der dazu konfiguriert ist, in oder auf eine atriale Herzkammer des Herzens (101) eines Patienten implantiert zu werden; und
einen ventrikulären Herzschrittmacher ohne Elektroden, vLP, (102b), der dazu konfiguriert ist, in oder auf eine ventrikuläre Herzkammer des Herzens (101) eines Patienten implantiert zu werden;
wobei jeder Herzschrittmacher ohne Elektroden, LP, des aLP (102a) und des vLP (102b) einen jeweiligen Sender (116, 722) beinhaltet, der es dem LP ermöglicht, Implantat-zu-Implantat-Nachrichten (i2i-Nachrichten) zu senden, einen jeweiligen Empfänger (120, 122) beinhaltet, der es dem LP ermöglicht, i2i-Nachrichten zu empfangen und eine jeweilige Stromquelle (114, 772) beinhaltet, die verwendet wird, um den Sender (116, 722) und den Empfänger (120, 122) des LP mit Strom zu versorgen;
wobei der aLP (102a) dazu konfiguriert ist, einen AAI-Betrieb bereitzustellen, und der vLP (102b) dazu konfiguriert ist, einen VVI-Betrieb bereitzustellen;
wobei, während der aLP (102a) den AAI-Betrieb bereitstellt, der aLP (102a) eine atriale Stimulation durchführt, wenn ein intrinsisches atriales Ereignis nicht innerhalb eines spezifizierten AA-Intervalls im Anschluss an ein zuvor stimuliertes oder intrinsisches atriales Ereignis erkannt wird, ein atriales Sensing durchführt und die atriale Stimulation unterdrückt, wenn das intrinsische atriale Ereignis innerhalb des spezifizierten AA-Intervalls im Anschluss an das zuvor stimulierte oder intrinsische atriale Ereignis erkannt wird; und
wobei, während der vLP (102b) den VVI-Betrieb bereitstellt, der vLP (102b) eine ventrikuläre Stimulation durchführt, wenn ein intrinsisches ventrikuläres Ereignis nicht innerhalb eines spezifizierten VV-Intervalls im Anschluss an ein zuvor stimuliertes oder intrinsisches ventrikuläres Ereignis erkannt wird, ein ventrikuläres Sensing durchführt und die ventrikuläre Stimulation unterdrückt, wenn das intrinsische ventrikuläre Ereignis innerhalb des spezifizierten VV-Intervalls im Anschluss an das zuvor stimulierte oder intrinsische ventrikuläre Ereignis erkannt wird, **dadurch gekennzeichnet, dass**
der aLP (102a) dazu konfiguriert ist, den AAI-Betrieb bereitzustellen, und der vLP (102b) dazu konfiguriert ist, den VVI-Betrieb bereitzustellen, während eines selben Zeitraums und unabhängig voneinander; und
während desselben Zeitraums, in dem der aLP (102a) den AAI-Betrieb bereitstellt und der vLP (102b) den VVI-Betrieb bereitstellt, unabhängig voneinander, der aLP (102a) dazu konfiguriert ist, auf das Senden von i2i-Nachrichten, die dazu vorgesehen sind, verwendet zu werden, um den Betrieb des vLP (102b) mit dem aLP (102a) zu koordinieren, zu verzichten, und der vLP (102b) dazu konfiguriert ist, auf das Senden von i2i-Nachrichten, die dazu vorgesehen sind, verwendet zu werden, um den Betrieb des aLP (102a) mit dem vLP (102b) zu koordinieren, zu verzichten.

2. System (100) nach einem der Ansprüche 1, wobei:
das spezifizierte AA-Intervall kürzer ist als das spezifizierte VV-Intervall, um zu bewirken, dass eine atriale Frequenz der durch den aLP (102a) durchgeführten atrialen Stimulation schneller ist als eine ventrikuläre Frequenz der durch den vLP (102b) durchgeführten ventrikulären Stimulation.

3. System (100) nach einem der Ansprüche 1 bis 2, wobei:
der aLP (102a) einen Sensor (152, 154) beinhaltet, der es dem aLP (102a) ermöglicht, ein Aktivitätsniveau eines Patienten zu überwachen, in den der aLP (102a) und der vLP (102b) implantiert sind;
jeder LP des aLP (102a) und des vLP (102b) eine jeweilige Steuerung (112, 720) beinhaltet;
die Steuerung (112, 720) des aLP (102a) dazu konfiguriert ist, eine atriale Frequenz der durch den aLP (102a) durchgeführten atrialen Stimulation durch Anpassen des spezifizierten AA-Intervalls basierend auf dem Aktivitätsniveau des Patienten, das durch Verwenden des Sensors (152, 154) des aLP (102a) erkannt wird, während der aLP (102a) den AAI-Betrieb bereitstellt, anzupassen; und
die Steuerung (112, 720) des vLP (102b) dazu konfiguriert ist, eine ventrikuläre Frequenz der durch den vLP (102b) durchgeführten ventrikulären Stimulation nicht anzupassen, während der vLP (102b) den VVI-Betrieb bereitstellt.

4. System (100) nach einem der Ansprüche 1 bis 3, wobei:
der aLP (102a) einen Sensing-Verstärker (132, 744) beinhaltet, der dazu konfiguriert ist, ein atriales Elektrogramm, aEGM, wahrzunehmen, das von dem aLP (102a) verwendet wird, um intrinsische atriale Ereignisse zu erkennen;
der vLP (102b) einen Sensing-Verstärker (132, 744) beinhaltet, der dazu konfiguriert ist, ein ventrikuläres Elektrogramm, vEGM, wahrzunehmen, das von dem vLP (102b) verwendet wird, um intrinsische ventrikuläre Ereignisse zu erkennen;
der aLP (102a) eine Steuerung (112, 720) beinhaltet, die kommunikativ mit dem Sensing-Verstärker (132, 744) des aLP (102a) gekoppelt ist;
der vLP (102b) eine Steuerung (112, 720) beinhaltet, die kommunikativ mit dem Sensing-Verstärker (132, 744) des vLP (102b) gekoppelt ist; und
die Steuerung (112, 720) des vLP (102b) dazu konfiguriert ist, auf mögliches Übersprechen zu überwachen, das von dem aLP (102a) verursacht werden kann, der die atriale Stimulation durchführt, während der vLP (102b) den VVI-Betrieb bereitstellt, und Bereitstellen eines Übersprechschutzes für einen ersten Übersprechschutz-Zeitraum einzuleiten, als Reaktion auf Erkennen des möglichen Übersprechens, das von dem aLP (102a) verursacht werden kann, der die atriale Stimulation durchführt.

5. System (100) nach Anspruch 4, wobei:
die Steuerung (112, 720) des aLP (102a) dazu konfiguriert ist, auf mögliches Übersprechen zu überwachen, das von dem vLP (102b) verursacht werden kann, der die ventrikuläre Stimulation durchführt, während der aLP (102a) den AAI-Betrieb bereitstellt, und Bereitstellen eines Übersprechschutzes für einen ersten Übersprechschutz-Zeitraum einzuleiten, als Reaktion auf Erkennen des möglichen Übersprechens, das von dem vLP (102b) verursacht werden kann, der die ventrikuläre Stimulation durchführt.

6. System (100) nach einem der Ansprüche 1 bis 5, wobei:
jeder Herzschrittmacher ohne Elektroden, LP, des aLP (102a) und des vLP (102b) einen jeweiligen Sender (116, 722) beinhaltet, der es dem LP ermöglicht, Implantat-zu-Implantat-Nachrichten (i2i-Nachrichten) zu senden, einen jeweiligen Empfänger (120, 122) beinhaltet, der es dem LP ermöglicht, i2i-Nachrichten zu empfangen und eine jeweilige Batterie (114, 772) beinhaltet, die verwendet wird, um den Sender (116, 722) und den Empfänger (120, 122) des LP mit Strom zu versorgen;
der aLP (102a) eine Steuerung (112, 720) beinhaltet, die kommunikativ mit dem Sender (116, 722) und dem Empfänger (120, 122) desselben gekoppelt ist und von dessen Batterie (114, 772) mit Strom versorgt wird;
der vLP (102b) eine Steuerung (112, 720) beinhaltet, die kommunikativ mit dem Sender (116, 722) und dem Empfänger (120, 122) desselben gekoppelt ist und von dessen Batterie (114, 772) mit Strom versorgt wird;
die Steuerung (112, 720) des vLP (102b) dazu konfiguriert ist, zu bestimmen, ob ein erstes spezifiziertes Kriterium erfüllt ist oder nicht, auf Verwenden des Senders (116, 722) des vLP (102b), um i2i-Nachrichten zu senden, die dazu vorgesehen sind, von dem aLP (102a) verwendet zu werden, um den Betrieb mit dem vLP (102b) während eines ersten Zeitraums, wenn das erste spezifizierte Kriterium nicht erfüllt ist, zu verzichten, und den Sender (116, 722) des vLP (102b) zu veranlassen, eine Modusumschalt-i2i-Nachricht als Reaktion darauf zu senden, dass das erste spezifizierte Kriterium erfüllt ist; und
die Modusumschalt-i2i-Nachricht, wenn sie von dem aLP (102a) erfolgreich empfangen und bestätigt wurde, den aLP (102a) und den vLP (102b) veranlasst, kollektiv einen koordinierten Zweikammerbetrieb bereitzustellen.

7. System (100) nach Anspruch 6, wobei die Steuerung (112, 720) des aLP (102a) zu Folgendem konfiguriert ist:
mindestens einen Teil des Empfängers (120, 122) des aLP (102a), der es dem aLP (102a) ermöglicht, eine oder mehrere i2i-Nachrichten von dem vLP (102b) zu empfangen, normalerweise zu deaktivieren, um dadurch Strom der Batterie (114, 772) des aLP (102a) im Vergleich zu dem Fall zu sparen, in dem der mindestens der Teil des Empfängers (120, 122) des aLP (102a) aktiviert ist;
den mindestens der Teil des Empfängers (120, 122) des aLP (102a), der es dem aLP (102a) ermöglicht, eine oder mehrere i2i-Nachrichten von dem vLP (102b) zu empfangen, von Zeit zu Zeit temporär zu aktivieren, um es dadurch dem aLP (102a) zu ermöglichen, auf die Modusumschalt-i2i-Nachricht zu überwachen, die den aLP (102a) dazu veranlasst, den Modus von dem Bereitstellen des AAI-Betriebs auf das kollektive Bereitstellen mit dem vLP (102b) des koordinierten Zweikammerbetriebs umzuschalten; und
den aLP (102a) dazu zu veranlassen, den Modus von dem Bereitstellen des AAI-Betriebs auf das kollektive Bereitstellen mit dem vLP (102b) des koordinierten Zweikammerbetriebs umzuschalten, als Reaktion auf das Empfangen und Bestätigen der Modusumschalt-i2i-Nachricht von dem vLP (102b) durch den aLP (102a).

8. System (100) nach einem der Ansprüche 6 bis 7, wobei:
das erste spezifizierte Kriterium, in Bezug auf das die Steuerung (112, 720) des vLP (102b) dazu konfiguriert ist, zu bestimmen, ob es erfüllt ist oder nicht, umfasst, dass der vLP (102b) mindestens eine erste spezifizierte Schwellenwertmenge ventrikulärer Stimulation innerhalb einer ersten spezifizierten Dauer bereitgestellt hat.

9. System (100) nach Anspruch 8, wobei die Steuerung (112, 720) des vLP (102b) zu Folgendem konfiguriert ist:
Bestimmen, ob ein zweites spezifiziertes Kriterium erfüllt ist oder nicht, nachdem der vLP (102b) den Modus von dem Bereitstellen des VVI-Betriebs auf das kollektive Bereitstellen mit dem aLP (102a) des koordinierten Zweikammerbetriebs umgeschaltet hat; und
während eines dritten Zeitraums, der als Reaktion darauf beginnt, dass das zweite spezifizierte Kriterium erfüllt ist, Verwenden des Senders (116, 722) des vLP (102b), um eine weitere Modusumschalt-i2i-Nachricht an den aLP (102a) zu senden, die, wenn sie von dem aLP (102a) erfolgreich empfangen und bestätigt wurde, den aLP (102a) veranlasst, den Modus von dem kollektiven Bereitstellen mit dem vLP (102b) des koordinierten Zweikammerbetriebs darauf umzuschalten, dass der aLP (102a) wieder den AAI-Betrieb unabhängig von dem vLP (102b) bereitstellt, und den vLP (102b) veranlasst, den Modus von dem kollektiven Bereitstellen mit dem aLP (102a) des koordinierten Zweikammerbetriebs darauf umzuschalten, dass der vLP (102b) wieder den VVI-Betrieb unabhängig von dem aLP (102a) bereitstellt.

10. System (100) nach Anspruch 9, wobei:
das zweite spezifizierte Kriterium, in Bezug auf das die Steuerung (112, 720) des vLP (102b) dazu konfiguriert ist, zu bestimmen, ob es erfüllt ist oder nicht, umfasst, dass der vLP (102b) weniger als eine zweite spezifizierte Schwellenwertmenge ventrikulärer Stimulation innerhalb einer zweiten spezifizierten Dauer bereitgestellt hat.

11. System (100) nach Anspruch 10, wobei:
während der aLP den AAI-Betrieb bereitstellt und der vLP den VVI-Betrieb bereitstellt, unabhängig voneinander, die Steuerung (112, 720) des ALP (102a) dazu konfiguriert ist, auf ein drittes spezifisches Kriterium zu überwachen, das, wenn es erkannt wird den Sender (116, 722) des aLP (102a) dazu veranlasst, eine i2i-Nachricht an den vLP (102b) oder an ein drittes IMD zu senden, das als Kommunikationsschnittstelle für die LPs fungiert, wodurch der vLP (102b) angewiesen wird, seine Basis-Stimulationsfrequenz oder sein Basis-VV-Intervall zu ändern.

12. System (100) nach Anspruch 11, wobei:
das dritte spezifizierte Kriterium, in Bezug auf das die Steuerung (112, 720) des aLP (102a) dazu konfiguriert ist, zu bestimmen, ob es erfüllt ist oder nicht, umfasst, dass der aLP (102a) erkannt hat, dass die atriale Frequenz einen spezifizierten Schwellenwert überschreitet.

13. System (100) nach einem der Ansprüche 6 bis 11, wobei der koordinierte Zweikammerbetrieb einen DDD-Betrieb umfasst.

14. System (100) nach einem der Ansprüche 6 bis 12, wobei der koordinierte Zweikammerbetrieb einen von einem VDD-, DDI-, VDI- oder DOO-Betrieb umfasst.

## Revendications

1. Système de stimulateur cardiaque sans fil, LP, à double chambre (100), comprenant :
un stimulateur cardiaque sans fil auriculaire, aLP, (102a) configuré pour être implanté dans ou sur une chambre cardiaque auriculaire du cœur d'un patient (101) ; et
un stimulateur cardiaque sans fil ventriculaire, vLP, (102b) configuré pour être implanté dans ou sur une chambre cardiaque ventriculaire du cœur du patient (101) ;
chaque stimulateur cardiaque sans fil, LP, de l'aLP (102a) et du vLP (102b), inclut un émetteur (116, 722) respectif qui permet au LP de transmettre des messages d'implant à implant (i2i), inclut un récepteur (120, 122) respectif qui permet au LP de recevoir des messages i2i, et inclut une source d'alimentation (114, 772) respective utilisée pour alimenter l'émetteur (116, 722) et le récepteur (120, 122) du LP ;
dans lequel l'aLP (102a) est configuré pour assurer un fonctionnement AAI, et le vLP (102b) est configuré pour assurer un fonctionnement VVI ;
dans lequel tandis que l'aLP (102a) assure le fonctionnement AAI, l'aLP (102a) effectue une stimulation auriculaire lorsqu'un événement auriculaire intrinsèque n'est pas détecté dans un intervalle AA spécifié suivant un événement auriculaire stimulé ou intrinsèque précédent, effectue une détection auriculaire, et inhibe la stimulation auriculaire lorsque l'événement auriculaire intrinsèque est détecté dans l'intervalle AA spécifié suivant l'événement auriculaire stimulé ou intrinsèque précédent ; et
dans lequel tandis que le vLP (102b) assure le fonctionnement VVI, le vLP (102b) effectue une stimulation ventriculaire lorsqu'un événement ventriculaire intrinsèque n'est pas détecté dans un intervalle VV spécifié suivant un événement ventriculaire stimulé ou intrinsèque précédent, effectue une détection ventriculaire, et inhibe la stimulation ventriculaire lorsque l'événement ventriculaire intrinsèque est détecté dans l'intervalle VV spécifié suivant l'événement ventriculaire stimulé ou intrinsèque précédent, **caractérisé en ce que**
l'aLP (102a) est configuré pour assurer le fonctionnement AAI, et le vLP (102b) est configuré pour assurer le fonctionnement VVI, pendant une même période de temps et indépendamment l'un de l'autre ; et
pendant la même période de temps que l'aLP (102a) assure le fonctionnement AAI et le vLP (102b) assure le fonctionnement VVI indépendamment l'un de l'autre, l'aLP (102a) est configuré pour s'abstenir de transmettre tout message i2i destiné à être utilisé pour coordonner le fonctionnement du vLP (102b) avec l'aLP (102a), et le vLP (102b) est configuré pour s'abstenir de transmettre tout message i2i destiné à être utilisé pour coordonner le fonctionnement de l'aLP (102a) avec le vLP (102b).

2. Système (100) selon l'une quelconque des revendications 1, dans lequel :
l'intervalle AA spécifié est plus court que l'intervalle VV spécifié pour amener une fréquence auriculaire de la stimulation auriculaire effectuée par l'aLP (102a) à être plus rapide qu'une fréquence ventriculaire de la stimulation ventriculaire effectuée par le vLP (102b).

3. Système (100) selon l'une quelconque des revendications 1 à 2, dans lequel :
l'aLP (102a) inclut un capteur (152, 154) qui permet à l'aLP (102a) de surveiller un niveau d'activité d'un patient à l'intérieur duquel l'aLP (102a) et le vLP (102b) sont implantés ;
chaque LP, de l'aLP (102a) et du vLP (102b), inclut un dispositif de commande (112, 720) respectif ;
le dispositif de commande (112, 720) de l'aLP (102a) est configuré pour ajuster une fréquence auriculaire de la stimulation auriculaire effectuée par l'aLP (102a) en ajustant l'intervalle AA spécifié sur la base du niveau d'activité du patient tel que détecté en utilisant le capteur (152, 154) de l'aLP (102a) tandis que l'aLP (102a) assure le fonctionnement AAI ; et
le dispositif de commande (112, 720) du vLP (102b) est configuré pour ne pas ajuster une fréquence ventriculaire de la stimulation ventriculaire effectuée par le vLP (102b) tandis que le vLP (102b) assure le fonctionnement VVI.

4. Système (100) selon l'une quelconque des revendications 1 à 3, dans lequel :
l'aLP (102a) inclut un amplificateur de détection (132, 744) configuré pour détecter un électrogramme auriculaire, aEGM, utilisé par l'aLP (102a) pour détecter des événements auriculaires intrinsèques ;
le vLP (102b) inclut un amplificateur de détection (132, 744) configuré pour détecter un électrogramme ventriculaire, vEGM, utilisé par le vLP (102b) pour détecter des événements ventriculaires intrinsèques ;
l'aLP (102a) inclut un dispositif de commande (112, 720) qui est couplé de manière communicative à l'amplificateur de détection (132, 744) de l'aLP (102a) ;
le vLP (102b) inclut un dispositif de commande (112, 720) qui est couplé de manière communicative à l'amplificateur de détection (132, 744) du vLP (102b) ; et
le dispositif de commande (112, 720) du vLP (102b) est configuré pour surveiller une possible diaphonie qui peut être causée par l'aLP (102a) effectuant la stimulation auriculaire, tandis que le vLP (102b) assure le fonctionnement VVI, et initier la fourniture d'une protection contre la diaphonie pendant une première période de protection contre la diaphonie, en réponse à la détection d'une possible diaphonie qui peut être causée par l'aLP (102a) effectuant la stimulation auriculaire.

5. Système (100) selon la revendication 4, dans lequel :
le dispositif de commande (112, 720) de l'aLP (102a) est configuré pour surveiller une possible diaphonie qui peut être causée par le vLP (102b) effectuant la stimulation ventriculaire, tandis que l'aLP (102a) assure le fonctionnement AAI, et initier la fourniture d'une protection contre la diaphonie pendant une seconde période de protection contre la diaphonie, en réponse à la détection d'une possible diaphonie qui peut être causée par le vLP (102b) effectuant la stimulation ventriculaire.

6. Système (100) selon l'une quelconque des revendications 1 à 5, dans lequel :
chaque stimulateur cardiaque sans fil, LP, de l'aLP (102a) et du vLP (102b), inclut un émetteur (116, 722) respectif qui permet au LP de transmettre des messages d'implant à implant (i2i), inclut un récepteur (120, 122) respectif qui permet au LP de recevoir des messages i2i, et inclut une batterie (114, 772) respective utilisée pour alimenter l'émetteur (116, 722) et le récepteur (120, 122) du LP ;
l'aLP (102a) inclut un dispositif de commande (112, 720) qui est couplé de manière communicative à l'émetteur (116, 722) et au récepteur (120, 122) de celui-ci et est alimenté par la batterie (114, 772) de celui-ci ;
le vLP (102b) inclut un dispositif de commande (112, 720) qui est couplé de manière communicative à l'émetteur (116, 722) et au récepteur (120, 122) de celui-ci et est alimenté par la batterie (114, 772) de celui-ci ;
le dispositif de commande (112, 720) du vLP (102b) est configuré pour déterminer si un premier critère spécifié est satisfait ou non, s'abstenir d'utiliser l'émetteur (116, 722) du vLP (102b) pour transmettre tout message i2i qui est destiné à être utilisé par l'aLP (102a) pour coordonner le fonctionnement avec le vLP (102b) pendant une première période de temps lorsque le premier critère spécifié n'est pas satisfait, et amener l'émetteur (116, 722) du vLP (102b) à transmettre un message i2i de type commutation de mode en réponse au fait que le premier critère spécifié est satisfait ; et
le message i2i de type commutation de mode, s'il est reçu avec succès par et s'il en est accusé réception par l'aLP (102a), amène l'aLP (102a) et le vLP (102b) à assurer collectivement un fonctionnement à double chambre coordonné.

7. Système (100) selon la revendication 6, dans lequel le dispositif de commande (112, 720) de l'aLP (102a) est configuré pour :
désactiver normalement au moins une portion du récepteur (120, 122) de l'aLP (102a) qui permet à l'aLP (102a) de recevoir un ou plusieurs messages i2i provenant du vLP (102b) pour ainsi économiser l'énergie de la batterie (114, 772) de l'aLP (102a) par rapport au cas où l'au moins une portion du récepteur (120, 122) de l'aLP (102a) serait activée ;
activer temporairement de temps à autre l'au moins une portion du récepteur (120, 122) de l'aLP (102a), qui permet à l'aLP (102a) de recevoir un ou plusieurs messages i2i provenant du vLP (102b), pour ainsi permettre à l'aLP (102a) de surveiller le message i2i de type commutation de mode qui amène l'aLP (102a) à commuter de mode, passant de la fourniture du fonctionnement AAI à la fourniture collective avec le vLP (102b) du fonctionnement à double chambre coordonné ; et
amener l'aLP (102a) à commuter de mode, passant de la fourniture du fonctionnement AAI à la fourniture collective avec le vLP (102b) du fonctionnement à double chambre coordonné, en réponse à la réception et à l'accusé de réception par l'aLP (102a) du message i2i de type commutation de mode provenant du vLP (102b).

8. Système (100) selon l'une quelconque des revendications 6 à 7, dans lequel :
le premier critère spécifié, que le dispositif de commande (112, 720) du vLP (102b) est configuré pour déterminer s'il est satisfait ou non, comprend le fait que le vLP (102b) a fourni au moins une première quantité de seuil spécifiée de stimulation ventriculaire d'ans une première durée spécifiée.

9. Système (100) selon la revendication 8, dans lequel le dispositif de commande (112, 720) du vLP (102b) est configuré pour :
déterminer si un deuxième critère spécifié est satisfait ou non, après que le vLP (102b) a commuté de mode, passant de la fourniture du fonctionnement VVI à la fourniture collective avec l'aLP (102a) du fonctionnement à double chambre coordonné ; et
pendant une troisième période de temps qui commence en réponse au fait que le deuxième critère spécifié est satisfait, utiliser l'émetteur (116, 722) du vLP (102b) pour transmettre un autre message i2i de type commutation de mode à l'aLP (102a), lequel, s'il est reçu avec succès par et s'il en est accusé réception par l'aLP (102a), amène l'aLP (102a) à commuter de mode, passant de la fourniture collective avec le vLP (102b) du fonctionnement à double chambre coordonné à la fourniture à nouveau par l'aLP (102a) du fonctionnement AAI indépendamment du vLP (102b), et amène le vLP (102b) à commuter de mode, passant de la fourniture collective avec l'aLP (102a) du fonctionnement à double chambre coordonné à la fourniture à nouveau par le vLP (102b) du fonctionnement VVI indépendamment de l'aLP (102a).

10. Système (100) selon la revendication 9, dans lequel :
le deuxième critère spécifié, que le dispositif de commande (112, 720) du vLP (102b) est configuré pour déterminer s'il est satisfait ou non, comprend le fait que le vLP (102b) a fourni moins d'une deuxième quantité de seuil spécifiée de stimulation ventriculaire dans une deuxième durée spécifiée.

11. Système (100) selon la revendication 10, dans lequel :
tandis que l'aLP assure le fonctionnement AAI et que le vLP assure le fonctionnement VVI, indépendamment l'un de l'autre, le dispositif de commande (112, 720) de l'aLP (102a) est configuré pour surveiller un troisième critère spécifique qui, lorsqu'il est détecté, amène l'émetteur (116, 722) de l'aLP (102a) à envoyer un message i2i au vLP (102b), ou à un troisième IMD qui agit comme un concentrateur de communication pour les LP, donnant l'instruction au vLP (102b) de modifier sa fréquence de stimulation de base ou son intervalle VV de base.

12. Système (100) selon la revendication 11, dans lequel :
le troisième critère spécifié, que le dispositif de commande (112, 720) de l'aLP (102a) est configuré pour déterminer s'il est satisfait ou non, comprend le fait que l'aLP (102a) a détecté que la fréquence auriculaire dépasse un seuil spécifié.

13. Système (100) selon l'une quelconque des revendications 6 à 11, dans lequel le fonctionnement à double chambre coordonné comprend un fonctionnement DDD.

14. Système (100) selon l'une quelconque des revendications 6 à 12, dans lequel le fonctionnement à double chambre coordonné comprend l'un parmi un fonctionnement VDD, DDI, VDI ou DOO.
